# EUROPEAN PATENT APPLICATION

(11) **EP 4 546 009 A1**
(43) Date of publication of application: **30.04.2025**
(21) Application number: 23826928.6
(22) Date of filing: 31.05.2023
(51) Int. Cl.: G01S 11/02

(54) **MOVING BODY, AND DISTANCE ESTIMATING METHOD**

(30) Priority: 21.06.2022 JP 2022099655
(71) Applicant: Panasonic Holdings Corporation, Osaka, 571-8501 (JP)
(72) Inventor: KOGA, Hisao, Osaka 571-0057 (JP)
(74) Representative: Eisenführ Speiser
(86) International application number: PCT/JP2023/020225
(87) International publication number: WO 2023/248741

(57) **Abstract**

A moving body according to an example of the present disclosure is capable of moving through a plurality of different media, and comprises: a communication unit for performing wireless communication with an external device; an index acquiring unit for acquiring a transmission path characteristic between the communication unit and the external device; a selecting unit for selecting a profile corresponding to a medium in which the moving body exists; and a distance estimating unit for estimating a distance to the external device on the basis of the transmission path characteristic and the profile.

## Description

### Technical Field

The present disclosure relates to a moving body and a distance estimation method.

### Background Art

In the related art, there is a technique for calculating a straight line distance between communication devices from a communication characteristic between the communication devices (for example, see Patent Literature (hereinafter referred to as "PTL") 1). In PTL 1, a straight line distance between Bluetooth (registered trademark) enabled communication devices is calculated by utilizing received signal strength.

### Citation List

### Patent Literature

PTL 1
Japanese Patent Application Laid-Open No. 2014-095697

### Summary of Invention

In the technique described in PTL 1, however, it is difficult to calculate at least a distance between communication devices underwater. Thus, for a moving body that moves by communicating through a plurality of different media, such as midair and underwater, it is difficult to accurately calculate the distance between the moving body and another communication device since the communication environment varies depending on the medium.

One non-limiting and exemplary embodiment facilitates providing a moving body and a distance estimation method each capable of suppressing an error in distance estimation in any medium when the moving body moves through a plurality of different media.

A moving body according to an exemplary embodiment of the present disclosure is a moving body capable of moving through a plurality of different media, and includes: a communicator that performs wireless communication with an external apparatus; an index acquirer that acquires a transmission path characteristic between the moving body and the external apparatus; a selector that selects a profile corresponding to, among the plurality of different media, a medium in which the moving body is present; and a distance estimator that estimates a distance between the moving body and the external apparatus based on the transmission path characteristic and the profile.

A distance estimation method according to an exemplary embodiment of the present disclosure includes: acquiring, by a moving body capable of moving through a plurality of different media and performing wireless communication with an external apparatus, a transmission path characteristic between the moving body and the external apparatus; selecting, by the moving body, a profile corresponding to, among the plurality of different media, a medium in which the moving body is present; and estimating, by the moving body, a distance between the moving body and the external apparatus based on the transmission path characteristic and the profile.

It should be noted that general or specific embodiments may be implemented as a system, a method, an integrated circuit, a computer program, a storage medium, or any selective combination thereof.

According to an exemplary embodiment of the present disclosure, it is possible to suppress an error in distance estimation in any medium when a moving body moves through a plurality of different media since the distance between the moving body and an external apparatus is estimated based on a profile corresponding to a medium in which the moving body is present.

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

### Brief Description of Drawings

FIG. 1 illustrates an exemplary configuration of a wireless power/data transmission system according to Embodiment 1 of the present disclosure;
FIG. 2 illustrates an exemplary hardware configuration of an underwater drone according to Embodiment 1 of the present disclosure;
FIG. 3 illustrates an exemplary functional configuration of a CPU of the underwater drone according to Embodiment 1 of the present disclosure;
FIG. 4 is a flowchart illustrating an alignment example according to Embodiment 1 of the present disclosure;
FIG. 5 is a sequence diagram illustrating a tone map determination example according to Embodiment 1 of the present disclosure;
FIG. 6 illustrates an association of the distance (transmission-reception distance) between communication apparatuses with the PHY speed (PHY speed profile) according to Embodiment 1 of the present disclosure;
FIG. 7 illustrates step S402 in FIG. 4;
FIG. 8 is a flowchart illustrating an alignment example according to Embodiment 1 of the present disclosure;
FIG. 9 illustrates a circumstance in which an aircraft parking apron for landing is determined based on topology information according to Embodiment 1 of the present disclosure;
FIG. 10 illustrates an example of a multi-hop network according to Embodiment 1 of the present disclosure;
FIG. 11 is a flowchart illustrating an alignment example according to Embodiment 1 of the present disclosure;
FIG. 12 illustrates that the underwater drone moves so as to be connected directly below an aircraft parking apron at an end according to Embodiment 1 of the present disclosure;
FIG. 13A illustrates an example of a multi-hop network according to Embodiment 1 of the present disclosure;
FIG. 13B illustrates an example of a multi-hop network according to Embodiment 1 of the present disclosure;
FIG. 13C illustrates an example of a multi-hop network according to Embodiment 1 of the present disclosure;
FIG. 14 is a flowchart illustrating an operation example of the underwater drone according to Embodiment 1 of the present disclosure;
FIG. 15 is a flowchart illustrating an operation example of the underwater drone according to Embodiment 1 of the present disclosure;
FIG. 16 is a flowchart illustrating an operation example of the underwater drone according to Embodiment 1 of the present disclosure;
FIG. 17A is a flowchart illustrating an operation example of the underwater drone according to Embodiment 1 of the present disclosure;
FIG. 17B is the flowchart illustrating the operation example of the underwater drone according to Embodiment 1 of the present disclosure;
FIG. 18 illustrates an exemplary functional configuration of a CPU of an underwater drone according to Embodiment 2 of the present disclosure;
FIG. 19 illustrates PHY speed profiles in different communication media according to Embodiment 2 of the present disclosure;
FIG. 20 is a flowchart illustrating a mode determination example of Embodiment 2 of the present disclosure;
FIG. 21 is a flowchart illustrating a mode determination example of Embodiment 2 of the present disclosure;
FIG. 22 is a flowchart illustrating a mode determination example of Embodiment 2 of the present disclosure; and
FIG. 23 is a flowchart illustrating an operation example of a drone according to Embodiment 2 of the present disclosure.

### Description of Embodiments

Hereinafter, embodiments of the present disclosure will be described in detail with reference to the accompanying drawings as appropriate. However, a detailed description more than necessary may be omitted, such as a detailed description of an already well-known matter and a duplicated description for a substantially identical configuration, to avoid the following description becoming unnecessarily redundant and to facilitate understanding by those skilled in the art.

Note that, the accompanying drawings and the following description are provided for those skilled in the art to sufficiently understand the present disclosure, and are not intended to limit the subject matter described in the claims.

### (Embodiment 1)

### <Configuration of Wireless power/Data Transmission System>

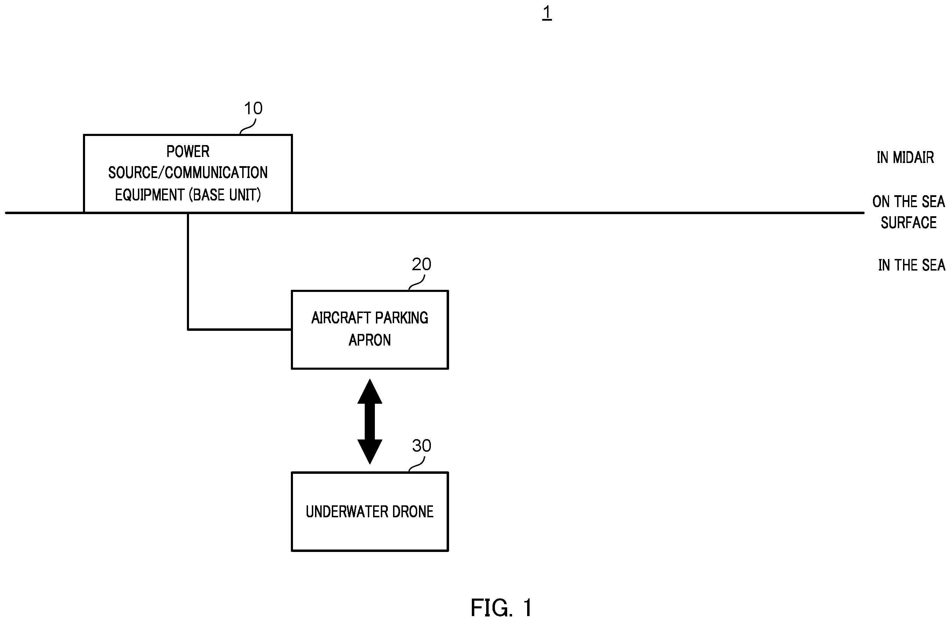

FIG. 1 illustrates an exemplary configuration of wireless power/data transmission system (or wireless power/data transmission network) 1 according to Embodiment 1 of the present disclosure.

As illustrated in FIG. 1, wireless power/data transmission system 1 is disposed on the water (for example, on the sea) and underwater (for example, in the sea). Wireless power/data transmission system 1 includes power source/communication equipment 10, aircraft parking apron (which may also be referred to as a charging (power supply) apparatus, a charging (power supply) station, or a station) 20, and underwater drone 30.

Power source/communication equipment 10 is provided, for example, in a vessel anchored on the sea (an example of: on the water). Power source/communication equipment 10 is connected to aircraft parking apron 20 with wire by using a communication line (including a power line) or the like. Power source/communication equipment 10 performs data communication with aircraft parking apron 20 and transmits electric power to aircraft parking apron 20. Power source/communication equipment 10 may also be referred to as a base unit, a master (communication) apparatus, or the like of wireless power/data transmission system 1.

Aircraft parking apron 20 has a wireless power transmission function and a wireless communication function. Aircraft parking apron 20 performs data communication with power source/communication equipment 10 and receives and supplies electric power transmitted from power source/communication equipment 10. In addition, aircraft parking apron 20 wirelessly communicates with underwater drone 30 and wirelessly transmits electric power to underwater drone 30. Note that, a plurality of aircraft parking aprons 20 may be present, aircraft parking aprons 20 may be connected to each other with wire or wirelessly. In this case, power transmission and data communication between aircraft parking aprons 20 may be the same as power transmission and data communication between power source/communication equipment 10 and aircraft parking apron 20, or power transmission and data communication between aircraft parking apron 20 and underwater drone 30.

Underwater drone 30 acquires various data related to work such as ocean exploration. Underwater drone 30 performs data communication with aircraft parking apron 20 and receives and supplies electric power transmitted from aircraft parking apron 20. For example, underwater drone 30 receives an instruction from power source/communication equipment 10 via aircraft parking apron 20, and transmits data (an image or the like) acquired during work, the charge state of underwater drone 30, and/or the like, to power source/communication equipment 10 via aircraft parking apron 20. Underwater drone 30 may also be referred to as, for example, an autonomous underwater vehicle (AUV), a remotely operated vehicle (ROV), an unmanned underwater vehicle (UUV), or the like. Underwater drone 30 is also capable of moving on the water surface, and may also be capable of moving in midair. A plurality of underwater drones 30 may also be present. Underwater drone 30 is an example of the "moving body" according to the present disclosure.

Note that, aircraft parking apron 20 may be realized by underwater drone 30. That is, underwater drone 30 may function as aircraft parking apron 20.

### [Configuration of Underwater Drone]

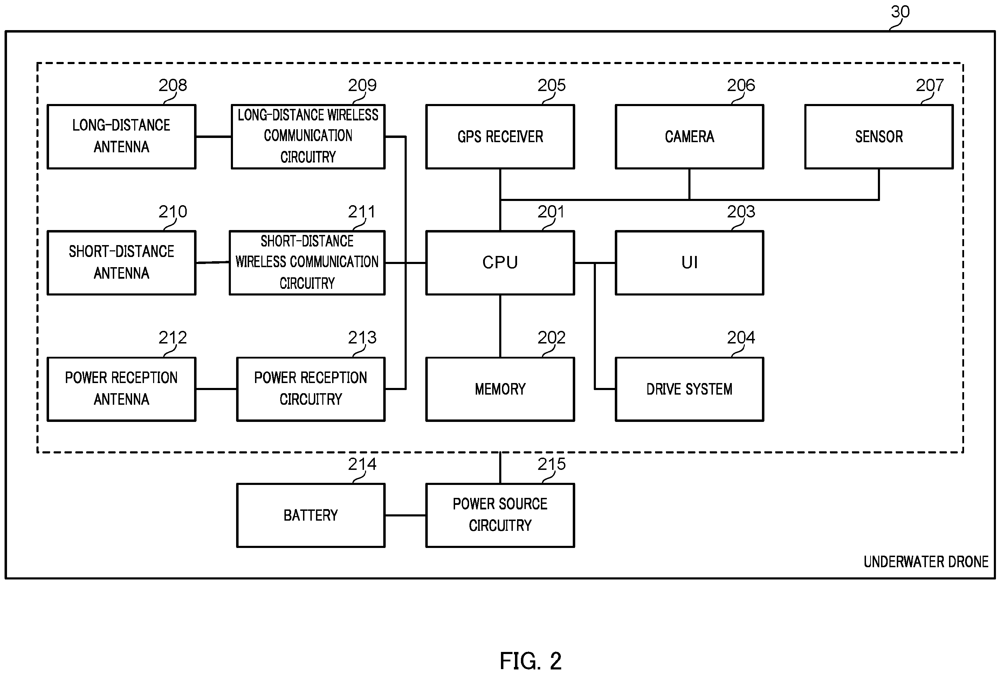

FIG. 2 illustrates an exemplary hardware configuration of underwater drone 30 according to the present embodiment.

As illustrated in FIG. 2, underwater drone 30 includes central processing unit (CPU) 201, memory 202, user interface (UI) 203, drive system 204, global positioning system (GPS) receiver 205, camera 206, sensor 207, long-distance antenna 208, long-distance wireless communication circuitry 209, short-distance antenna 210, short-distance wireless communication circuitry 211, power reception antenna 212, power reception circuitry 213, battery 214, and power source circuitry 215.

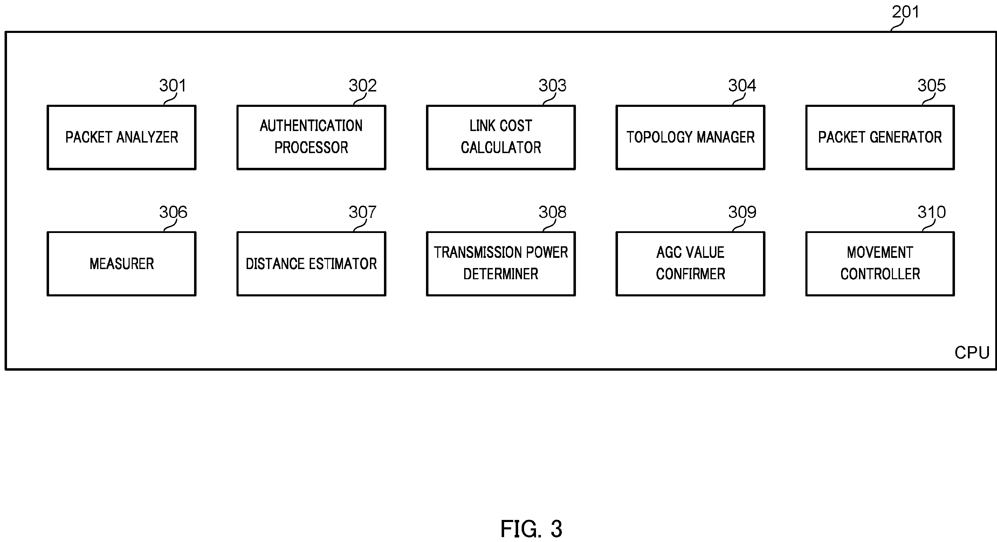

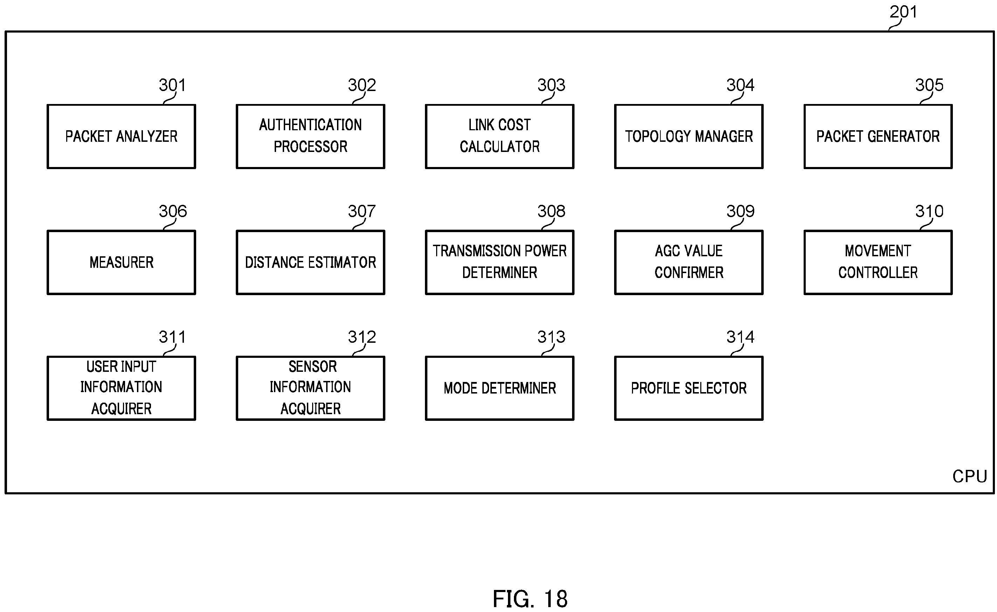

CPU 201 controls processing of each component, and executes processing as will be described with reference to FIG. 3 as well as FIG. 18 or the like.

Memory 202 stores (saves, holds) programs and data required for underwater drone 30 to operate, data generated by components of underwater drone 30, data received by underwater drone 30 from external apparatuses, and the like.

UI 203 includes an input apparatus that receives an operation on underwater drone 30, and an output apparatus that provides output of sound or the like.

Drive system 204 is a drive system for sailing underwater drone 30. The drive system includes, for example, a screw, a motor configured to turn the screw, and a battery configured to serve as a power source for the motor. Further, in a case where underwater drone 30 is capable of moving in midair, the drive system may include a propeller, a motor configured to turn the propeller, and a battery configured to serve as a power source for the motor.

GPS receiver 205 receives radio waves from a GPS satellite and measures the position of underwater drone 30 (for example, the latitude and longitude) by using the received radio waves.

Camera 206 is, for example, an omnidirectional camera, and is capable of capturing images in the front-rear, left-right, and up-down directions of underwater drone 30.

Sensor 207 includes a sensor configured to measure the state (acceleration, speed, and/or the like) of underwater drone 30, a sensor configured to measure the state of the surrounding environment of underwater drone 30, a sensor configured to measure the state of drive system 204 (such as a sensor configured to measure the state of a motor, a sensor configured to measure the state (including the remaining amount) of a battery, or the like), and/or the like. The sensor configured to measure the state of the surrounding environment includes a moisture sensor, a salinity sensor (salinometer), an atmospheric pressure sensor, a temperature sensor, a humidity sensor, an illuminance sensor, and/or the like.

Long-distance antenna 208 is an antenna for communicating via a mobile communication network supporting Long Term Evolution (LTE), New Radio (NR), and the like.

Long-distance wireless communication circuitry 209 is communication circuitry that performs transmission processing and reception processing in order to transmit and receive signals via the mobile communication network supporting LTE, NR, and the like, and long-distance antenna 208.

Short-distance antenna 210 is an antenna for communicating, for example, by using a frequency band such as 2 MHz to 100 MHz, with the aforementioned external apparatus, such as base unit 10, aircraft parking apron 20, and another underwater drone 30, which is located at a short distance with, for example, a transmission distance of 10 m or less.

Short-distance wireless communication circuitry 211 is communication circuitry that performs transmission processing and reception processing in order to transmit and receive signals via short-distance antenna 210.

Power reception antenna 212 is an antenna for receiving electric power from an external apparatus located at a short distance.

Power reception circuitry 213 is circuitry that receives electric power via power reception antenna 212.

Battery 214 supplies electric power to each component of underwater drone 30.

Power source circuitry 215 converts the electric power from battery 214 into an output power source supposed to be required for each component of underwater drone 30.

Note that, in the present embodiment and the other embodiment, a power supply coil and a communication coil in underwater drone 30 are disposed close to each other.

Underwater drone 30 performs short-distance communication with an external apparatus via short-distance antenna 210 and short-distance wireless communication circuitry 211 (referred to as a short-distance wireless processor). The external apparatus may be a fixed apparatus, such as aircraft parking apron 20 or a fixed-type wireless communication apparatus, or may be a moving body, such as an aerial drone, another underwater drone, or a vessel on the sea. Short-distance antenna 210 and short-distance wireless communication circuitry 211 are examples of the "communicator" according to the present disclosure.

Underwater drone 30 aligns with aircraft parking apron 20 and a vessel on the sea for wireless power supply and/or data communication, for example. Underwater drone 30 aligns with a fixed-type wireless communication apparatus for data communication, for example. Underwater drone 30 aligns with an aerial drone and another underwater drone for multi-hop formation (topology formation), for example.

Note that, power source/communication equipment 10 and aircraft parking apron 20 may also have the same configuration as underwater drone 30.

FIG. 3 illustrates an exemplary functional configuration of CPU 201.

CPU 201 includes packet analyzer 301, authentication processor 302, link cost calculator 303, topology manager 304, packet generator 305, measurer 306, distance estimator 307, transmission power determiner 308, automatic gain control (AGC) value confirmer 309, and movement controller 310. Note that, power source/communication equipment 10 and aircraft parking apron 20 also have the same functional configuration as underwater drone 30. In addition, hereinafter, power source/communication equipment 10, aircraft parking apron 20, and underwater drone 30 will be collectively referred to as communication apparatuses.

H packets, authentication packets, and normal packets (for example, data acquired by underwater drone 30) are inputted into CPU 201. H packets are packets that are simultaneously broadcast from one communication apparatus toward a connected communication apparatus prior to authentication processing that is performed in wireless power/data transmission network 1. Authentication packets are packets that are transmitted and received during the authentication processing.

Packet analyzer 301 analyzes the data structures of various packets (H packets, authentication packets, and normal packets) to be inputted to determine the types of packets, and distributes the forwarding destinations of the packets based on the determination results. For example, in a case where packet analyzer 301 determines that an inputted packet is an H packet, packet analyzer 301 outputs the H packet to link cost calculator 303. For example, in a case where packet analyzer 301 determines that an inputted packet is an authentication packet, packet analyzer 301 outputs the authentication packet to authentication processor 302. For example, in a case where packet analyzer 301 determines that an inputted packet is a normal packet, packet analyzer 301 outputs the normal packet to topology manager 304.

Authentication processor 302 uses an authentication packet inputted from packet analyzer 301 to execute authentication processing with a communication apparatus that has transmitted the authentication packet. Authentication processor 302 outputs, to topology manager 304, an authentication packet, which is a packet generated during the authentication processing, or a response to an authentication packet transmitted from another communication apparatus.

Link cost calculator 303 uses an H packet inputted from packet analyzer 301 to calculate a link cost indicating the reception quality of the H packet received by underwater drone 30 from the communication apparatus that has transmitted the H packet. Link cost calculator 303 writes (stores) the calculation result of the link cost into the data structure of the H packet. Further, each link cost value in a case where an H packet has been transmitted from base unit 10 is also stored in the H packet. Link cost calculator 303 outputs an H packet, in which the calculation result of the link-cost is stored, to topology manager 304.

Topology manager 304 manages (acquires) topology information indicating a topology (that is, communication apparatuses constituting wireless power/data transmission network 1 and the connection form thereof) generated by base unit 10, and outputs various packets by distributing the output destinations thereof (for example, packet generator 305) based on the topology information. The topology information is saved in topology manager 304 or memory 202 of each communication apparatus. Note that, topology manager 304 of base unit 10 generates (forms) a topology based on each link cost calculated by link cost calculator 303 of base unit 10 in response to H packets transmitted from one or more communication apparatuses, respectively, which are subjected to authentication processing, and provides topology information indicating the topology to another communication apparatus. Topology manager 304 is an example of the "acquirer" according to the present disclosure.

Packet generator 305 generates and outputs, based on an authentication packet or a normal packet inputted from topology manager 304, a packet for data communication to another communication apparatus to which the packet is transmitted, or an H packet for performing authentication processing.

Measurer 306 measures (calculates, acquires), by using a transmission path estimation technique, the physical layer communication speed (also referred to as the PHY speed) with the communication counterpart by performing transmission path estimation with the communication counterpart (by acquiring (calculating) an index value indicating a transmission path characteristic or the transmission path characteristic). Measurer 306 is an example of the "index acquirer" or the "index calculator" according to the present disclosure.

Distance estimator 307 estimates the distance between underwater drone 30 and the communication counterpart based on an association of the distance (transmission-reception distance) between communication apparatuses with the PHY speed (transmission path characteristic) (the association will be referred to as the PHY speed profile or simply the profile), as well as the PHY speed with the communication counterpart.

Transmission power determiner 308 determines and sets the transmission power for transmitting a signal (packet) to the communication counterpart.

AGC value confirmer 309 confirms the AGC value adjusted by AGC circuitry (not illustrated). The AGC circuitry sets, for example, an appropriate amplification degree according to the electric power of the received signal.

Movement controller 310 causes underwater drone 30 to move to a certain position or in a certain direction by controlling drive system 204 such that underwater drone 30 moves to the certain position or in the certain direction or underwater drone 30 stops at the certain position. For example, movement controller 310 causes underwater drone 30 to move or stop by controlling drive system 204 in the above-described manner based on the topology information acquired by topology manager 304, the PHY speed acquired by measurer 306, and/or the like.

### <Operations of Wireless Power/Data Transmission System>

Next, common operations of power source/communication equipment 10, aircraft parking apron 20, and underwater drone 30 will be described. Hereinafter, operations of underwater drone 30 will be described as an example, but power source/communication equipment 10 and aircraft parking apron 20 can also perform the same operations.

### [Authentication Processing]

An exemplary processing procedure of authentication processing that is performed between communication apparatuses (authentication processing that is performed between base unit 10 and aircraft parking apron 20 or underwater drone 30) will be described. This authentication processing is performed mainly by each authentication processor 302. The premise is that base unit 10 transmits an H packet to aircraft parking apron 20 or underwater drone 30, and that aircraft parking apron 20 or underwater drone 30 receives the H packet transmitted from base unit 10. Note that, hereinafter, operations of underwater drone 30 will be described as an example, but aircraft parking apron 20 can also perform the same operations.

Underwater drone 30 issues an authentication packet including a participation request frame for the base unit (base unit 10) of a network (for example, wireless power/data transmission network 1) in which underwater drone 30 wishes to participate, and transmits the authentication packet to base unit 10. When base unit 10 receives the authentication packet transmitted from underwater drone 30, base unit 10 sets a predetermined text string to generate an authentication packet, and transmits the authentication packet to underwater drone 30 that has made the request (underwater drone 30 that has issued the authentication packet).

Underwater drone 30 encodes the predetermined text string, which is included in the authentication packet transmitted from base unit 10, by using a unique key, which underwater drone 30 holds in memory 202 or the like in advance, and generates an authentication packet including the text string as the encoded output thereof, and transmits the authentication packet to base unit 10.

When base unit 10 receives the authentication packet transmitted from underwater drone 30, base unit 10 decodes the text string as the encoded output, which is included in the authentication packet, by using a unique key which is set in advance and is saved in memory 202 or the like. Further, base unit 10 determines whether the text string as the decoded output thereof matches the predetermined text string set in the above-described manner. In a case where base unit 10 determines that the text string as the decoded output matches the predetermined text string (in a case where base unit 10 determines that underwater drone 30 is allowed to participate in wireless power/data transmission network 1), base unit 10 encodes a network key specific to wireless power/data transmission network 1 with the unique key, and transmits the encoded network key to underwater drone 30.

Underwater drone 30 decodes the encoded network key transmitted from base unit 10 by using the unique key to acquire the network key. The acquisition of the network key means that underwater drone 30 is formally authenticated as a data communication destination for base unit 10. When underwater drone 30 performs data communication in the network (wireless power/data transmission network 1), underwater drone 30 performs the data communication by performing encoding or decoding by using the network key.

Note that, the authentication processing is, for example, processing based on the 4-way handshake defined in the standard of HD-PLC (High Definition Power Line Communication), but the authentication processing may be processing based on another scheme other than the above-described standard. For example, the authentication processing may be authentication processing based on Ghn (Gigabit Home Networking), which is a unified standard for high-speed wired network technology, or a scheme of Home Plug (Home Plug Power line Alliance), which is an industry organization for power-line carrier communication, or the like.

### [Topology Generation (Multi-hop Formation) Processing and Multi-Hop Transmission]

Next, topology generation and multi-hop transmission among base unit 10, aircraft parking apron 20, and underwater drone 30 will be described.

Base unit 10 simultaneously broadcasts H packets toward every communication apparatus. It is assumed only aircraft parking apron 20 can receive an H packet for this broadcast. Then, the authentication processing described above is performed between base unit 10 and aircraft parking apron 20. Thus, power transmission and data communication become possible between base unit 10 and aircraft parking apron 20.

Next, aircraft parking apron 20 simultaneously broadcasts H packets toward other communication apparatuses. When a communication apparatus is present that has been able to receive an H packet for this broadcast, the authentication processing described above is performed between the communication apparatus and base unit 10 via aircraft parking apron 20, with the result that data communication between the communication apparatus and base unit 10 via aircraft parking apron 20 becomes possible and power transmission between aircraft parking apron 20 and the communication apparatus becomes possible. The same processing is performed for the communication apparatus.

Here, it is assumed that underwater drone 30 approaches the above communication apparatus. Then, the above communication apparatus transmits an H packet toward underwater drone 30. It is assumed that underwater drone 30 has been able to receive an H packet for this transmission. Then, the authentication processing described above is performed between underwater drone 30 and aircraft parking apron 20 via the above communication apparatus and aircraft parking apron 20, with the result that data communication between underwater drone 30 and base unit 10 via the above communication apparatus and aircraft parking apron 20 becomes possible and power transmission between the above communication apparatus and the communication apparatus becomes possible.

### [First Alignment Example]

As described above, underwater drone 30 grasps the direction, the distance, and the like from the starting point by utilizing the functions of GPS receiver 205, sensor 207, and the like, and moves toward the destination. Further, underwater drone 30 also includes a mobile communication module (long-distance antenna 208, long-distance wireless communication circuitry 209), and when underwater drone 30 surfaces and is on the sea, underwater drone 30 performs bidirectional communication of control signals, performs communication using lateral waves that travel directly on the water surface, and grasps its position through GPS receiver 205. It is considered that underwater drone 30 grasps its landing location by using camera 206, performs, after landing at the landing location, charging wirelessly at the aircraft parking apron, and transmits and receives data by bidirectional communication. Note that, in the present specification, reaching an underwater destination is referred to as landing, and departing from an underwater destination is referred to as takeoff.

However, in a case where underwater drone 30 attempts to land at a destination by utilizing GPS, a sensor, a camera or the like, underwater drone 30 may not be able to land at an optimum location due to a problem of accuracy as described above, in which case the charging efficiency by wireless power transmission decreases, for example, when underwater drone 30 lands for charging. For example, considering a case where underwater drone 30 lands at a substantially circular aircraft parking apron as its destination, underwater drone 30 desirably lands near the center of the aircraft parking apron since the closer it is to the center of the aircraft parking apron, the faster the communication speed and the higher the charging efficiency (since the power supply coil and the communication coil are disposed close to each other).

Generally, in a case where two communication apparatuses perform short-distance wireless communication, the longer the distance (transmission-reception distance) between the communication apparatuses, the larger the attenuation amount of the transmission path, with the result that the communication speed becomes slower. The attenuation with respect to the distance underwater tends to be greater than that in the air, and the attenuation with respect to the distance in the sea tends to be much greater. **In** such cases, it is difficult to perform midair wireless communication at the landing location. For this reason, underwater drone 30 approaches its landing location to some extent, for example, by utilizing GPS receiver 205, sensor 207, and/or the like, and confirms the landing location by using camera 206 there. Subsequently, in the first alignment example, a transmission path estimation technique and the physical layer communication speed (PHY speed) are utilized in order to improve accuracy.

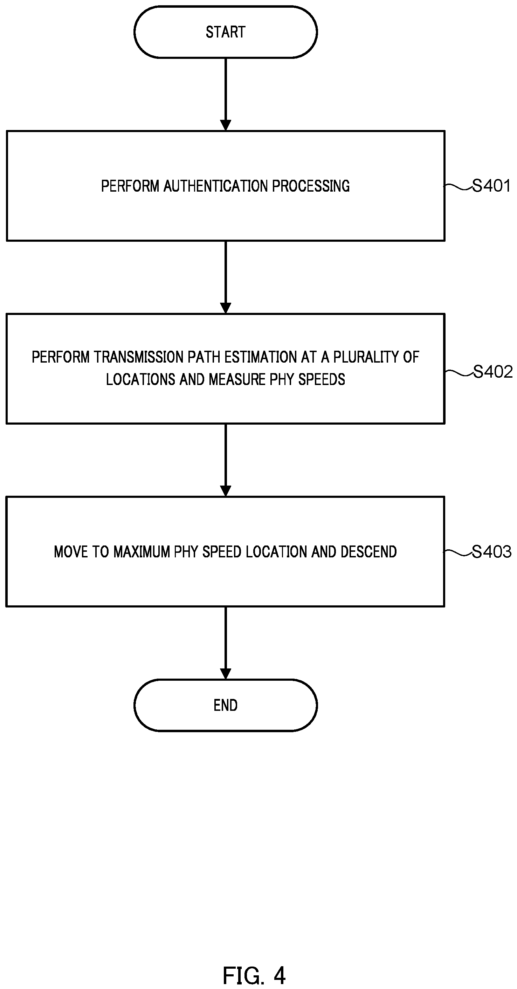

FIG. 4 is a flowchart illustrating an alignment example according to the present embodiment. This alignment may be performed, for example, for wireless power supply and/or data communication.

When underwater drone 30 approaches its landing location (aircraft parking apron 20), authentication processor 302 of underwater drone 30 performs the authentication processing described above with base unit 10 (via aircraft parking apron 20) in step S401.

When the authentication is successful, in step S402, underwater drone 30 moves in a planar manner in the horizontal direction under the control of movement controller 310. Thereby, measurer 306 of underwater drone 30 performs transmission path estimation at a plurality of locations and measures the PHY speeds. For example, the plurality of locations may be five locations consisting of a location, at which authentication processing is performed, and the vertices of an arbitrary square with sides having a predetermined length and with the aforementioned location as the center of gravity, but are not limited thereto. The transmission path estimation and the PHY speed estimation will be described later.

In step S403, underwater drone 30 moves to, among the plurality of locations, a location at which the PHY speed is the fastest (referred to as the maximum PHY speed location) under the control of movement controller 310, and descends from the location by a predetermined distance (for example, 10 cm).

Underwater drone 30 lands at aircraft parking apron 20 by repeating steps S402 and S403 thereafter. Note that, the number of repetitions may be determined in advance, or repetitions may be performed until the value of the distance between underwater drone 30 and aircraft parking apron 20 becomes equal to or less than a predetermined value or becomes less than the predetermined value as a result of the repetitions. Alternatively, in a case where the PHY speed at the current location becomes equal to or greater than a threshold, underwater drone 30 may stop repetitions. Further, the plurality of locations may be set such that the length of the sides of the square becomes shorter for each repetition. Thereafter, underwater drone 30 may remain at a position after repetitions, or may descend from the position to aircraft parking apron 20.

### [[Transmission Path Estimation Technique]]

A (transmission) tone map created by transmission path estimation is shared between communication apparatuses. Here, as the tone map, the optimum modulation level for each subcarrier of OFDM (Orthogonal Frequency Division Multiplexing) (for example, modulation level 5: 32 PAM (Pulse Amplitude Modulation), modulation level 4: 16 PAM, modulation level 3: 8 PAM, modulation level 2: 4 PAM, modulation level 1: 2 PAM, modulation level 0: none; the noise level becomes relatively larger with respect to the signal level as the modulation level becomes smaller) is set, communication is performed by optimizing the information amount for each carrier according to the signal-to-noise ratio (SN ratio). For example, the tone map may be a table in which the subcarriers of OFDM are associated with the modulation levels. In a case where it is assumed that the modulation levels are five stages in an above-described manner and, for example, are set for each of 360 transmission and reception carriers, a huge number (five to the power of 360) of key patterns can be configured, from which a tone map is selected. As described above, the tone map depends on the state of the transmission path and is often different for each transmission path, and thus, eavesdropping in a network becomes difficult.

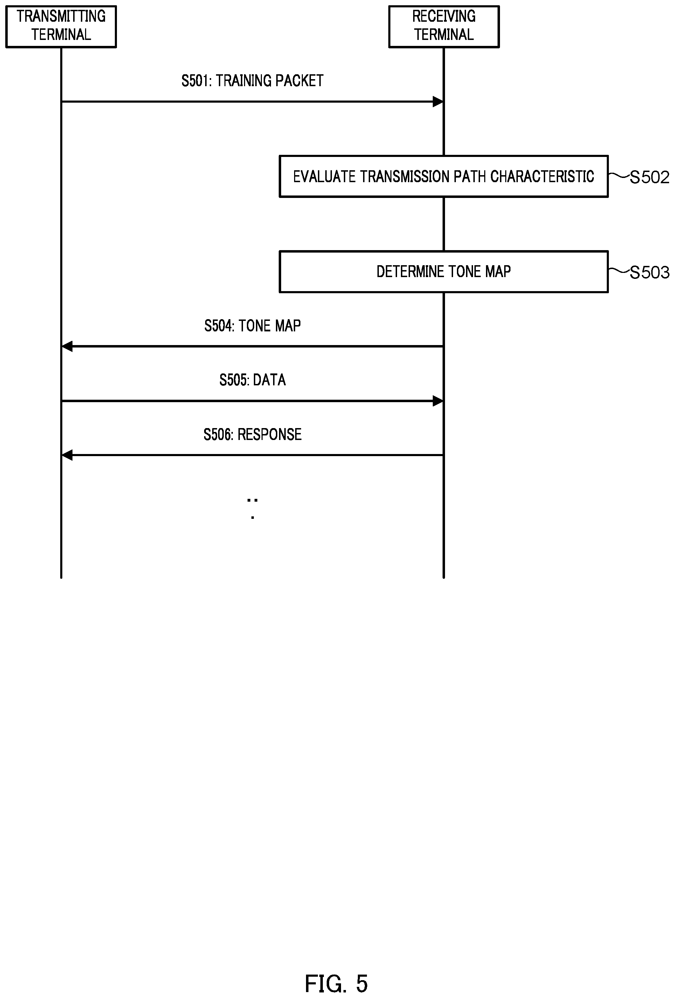

FIG. 5 is a sequence diagram illustrating a tone map determination example according to the present embodiment.

In step S501, the transmitting terminal (one communication apparatus) transmits a training packet to the receiving terminal (the other communication apparatus).

In step S502, the receiving terminal obtains an SN ratio characteristic by using the received training packet and performs transmission path characteristic evaluation.

In step S503, the receiving terminal determines (creates) a tone map.

In step S504, the receiving terminal indicates (transmits) the determined tone map to the transmitting terminal.

Subsequently, the transmitting terminal transmits data to the receiving terminal based on the tone map (step S505). In this transmission, high-speed transmission suitable for the transmission path is possible. Further, the receiving terminal returns a response (step S506). Subsequently, such transmission and reception may be further repeated.

Note that, an example in which a training packet is used for creating a tone map has been indicated above, but a normal data packet that is used in normal communication may be used instead of the training packet.

The transmitting terminal and the receiving terminal may be one of an external apparatus including aircraft parking apron 20 and underwater drone 30. For example, in the flow illustrated in FIG. 4, the receiving terminal is underwater drone 30 when the transmitting terminal is an external apparatus, whereas the receiving terminal is an external apparatus when the transmitting terminal is underwater drone 30. Accordingly, in the present embodiment and the other embodiment, a moving body such as underwater drone 30 may receive (acquire) a transmission path estimation result (a transmission path characteristic such as a tone map) transmitted by an external apparatus, which has performed transmission path estimation, to the moving body such as underwater drone 30, and thereby the moving body such as underwater drone 30 may measure the PHY speed based on the transmission path estimation result as described later.

Note that, the SN ratio, the tone map, and the PHY speed for each subcarrier are examples of the "index value indicating a transmission path characteristic" or the "transmission path characteristic" according to the present disclosure calculated or acquired from transmission and reception of signals to and from an external apparatus.

In the present embodiment and the other embodiment, the trigger to perform transmission path estimation may be a case where a moving body such as underwater drone 30 determines that the moving body is located near (above in midair, directly below, or the like) the destination (external apparatus) based on position information utilizing GPS, or may be a case where the moving body determines that the moving body is located near (above in midair, directly below, or the like) the destination (external apparatus) based on camera images. Additionally or alternatively, the trigger to perform transmission path estimation may be a case where a moving body such as underwater drone 30 receives or transmits a predetermined signal. The predetermined signal may be, for example, a signal for indicating the termination of the authentication processing described above. Further, the predetermined signal may be received or transmitted periodically (for example, once every few seconds, or the like), or may be started after the moving body determines that the moving body is located near (above in midair, directly below, or the like) the destination (external apparatus) based on position information utilizing GPS, camera images, and/or the like.

### [Relationship between Transmission-Reception Distance and PHY Speed]

The modulation levels included in a tone map as described above are associated with the PHY speeds. In addition, the associations of the modulation levels with the PHY speeds are saved in advance in memory 202 of underwater drone 30. For this reason, measurer 306 of underwater drone 30 can measure the PHY speed based on the acquired tone map and the association.

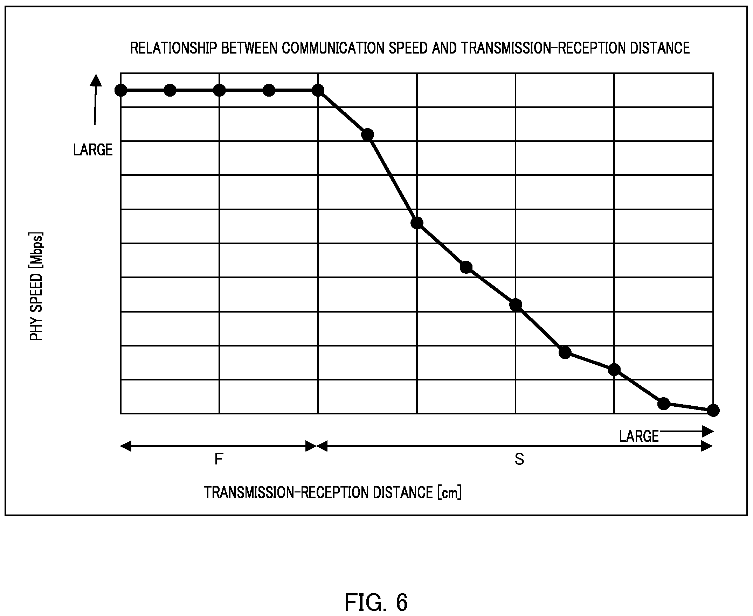

FIG. 6 illustrates an association of the distance (transmission-reception distance) between communication apparatuses with the PHY speed. Such an association is also saved in advance in memory 202 of underwater drone 30. As described above and as illustrated in the drawing, the longer the transmission-reception distance, the larger the attenuation amount of the transmission path, and the communication speed becomes slower. Distance estimator 307 of underwater drone 30 can estimate the transmission-reception distance based on the measured PHY speed and the association.

Given the above, underwater drone 30 can land at aircraft parking apron 20 by estimating the transmission-reception distance (the distance between underwater drone 30 and aircraft parking apron 20) based on the PHY speed.

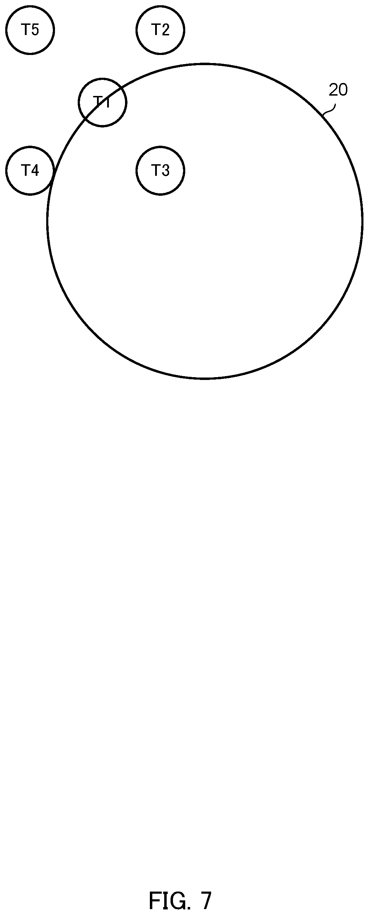

FIG. 7 illustrates step S402 in FIG. 4.

When underwater drone 30 approaches aircraft parking apron 20, underwater drone 30 first performs authentication processing with base unit 10 at location T1.

When the authentication is successful, underwater drone 30 performs transmission path estimation at locations T1 to T5 and measures the PHY speeds. Here, an arrangement of the PHY speeds in descending order results in, for example, the PHY speeds at location T3, location T1, location T4, location T2, and location T5.

Underwater drone 30 moves to the maximum PHY speed location T3 and descends from the maximum PHY speed location T3 by a predetermined distance.

As described above, when underwater drone 30 approaches its landing location, the authentication processing starts, and the guidance of the landing is performed. Thus, according to the present example, underwater drone 30 moves to the maximum PHY speed location close to the center of aircraft parking apron 20, and therefore can perform landing with high precision. In addition, since underwater drone 30 lands at a location close to the center, underwater drone 30 can be charged with high efficiency.

### [Second Alignment Example]

As illustrated on the left side of FIG. 6 (flat portion F), when underwater drone 30 and aircraft parking apron 20 are too close to each other, the PHY speeds measured at a plurality of locations may become saturated and only indicate values close to the upper limit in step S402 in FIG. 4, which may not lead to an improvement in accuracy. Accordingly, in order to prevent the saturation of the PHY speeds obtained from transmission path estimation at a plurality of locations, underwater drone 30 may perform, while confirming the AGC value or the like, power control in a case where it is determined that transmission power is large.

By confirming the AGC value, it is possible to grasp the specific extent of attenuation occurring in the transmission path. A large AGC value means that the amplification degree is large due to a large attenuation of the transmission path. In such an environment, the electric power of the received signal is so small that large amplification is required, that is, the transmission-reception distance becomes long. For example, as the attenuation of the transmission path is larger, it is necessary to increase the AGC value and the amplification degree to amplify the signal. In a case where the AGC value is small (that is, large amplification is not required), on the other hand, it is suggested that the electric power of the received signal is large.

Accordingly, in the case of the present example, when the AGC value becomes smaller (in other words, when the electric power of the received signal is large), it is controlled such that the transmission power decreases to relatively increase the attenuation of the transmission path. Thereby, it is possible to change the scale of the horizontal axis illustrated in FIG. 6 and to change from the evaluation using the left side of FIG. 6 (flat portion F) to the evaluation using the right side of FIG. 6 (slope portion S). Thus, it is possible to perform measurement with high accuracy even in a case where the transmission-reception distance decreases.

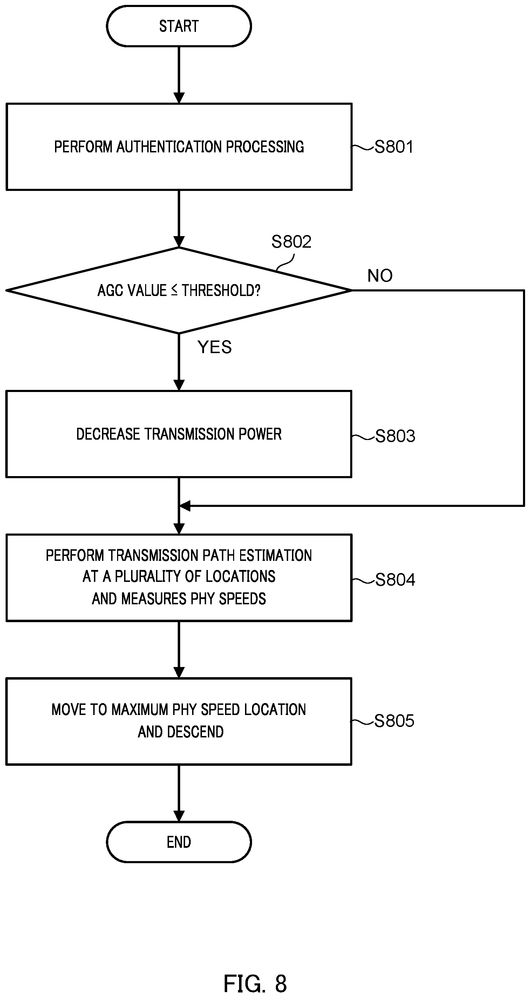

FIG. 8 is a flowchart illustrating an alignment example according to the present embodiment. This alignment may be performed, for example, for wireless power supply and/or data communication.

Step S801 is the same as step S401 in FIG. 4.

When the authentication is successful, in step S802, AGC value confirmer 309 of underwater drone 30 determines whether the AGC value is equal to or less than a threshold.

In a case where the AGC value is not equal to or less than the threshold (NO in step S802), the flow proceeds to step S804.

In a case where the AGC value is equal to or less than the threshold (YES in step S803), transmission power determiner 308 of underwater drone 30 determines and sets the transmission power in step S803 such that the set transmission power decreases. For example, transmission power determiner 308 determines and sets the transmission power whose value is a half, a quarter, of the like of the value of the original transmission power.

Steps S804 and S805 are the same as steps S402 and S403 in FIG. 4, respectively.

Underwater drone 30 lands at aircraft parking apron 20 by repeating steps S802 to S805 thereafter. Note that, the number of repetitions may be determined in advance, or repetitions may be performed until the distance between underwater drone 30 and aircraft parking apron 20 becomes equal to or less than a predetermined value or becomes less than the predetermined value as a result of the repetitions. Alternatively, in a case where the PHY speed at the current location becomes equal to or greater than a threshold, underwater drone 30 may stop repetitions. Further, the plurality of locations may be set such that the length of the sides of the square becomes shorter for each repetition. Thereafter, underwater drone 30 may remain at a position after repetitions, or may descend from the position to aircraft parking apron 20.

As described above, before transmission path estimation is performed at a plurality of locations, underwater drone 30 may confirm the AGC value and determine the transmission power (for example, determines the transmission power whose value is a half, a quarter, of the like of the value of the original transmission power in a case where the AGC value is equal to or less than the threshold, and otherwise determines the transmission power whose value remains the same as the original value), and may perform transmission path estimation at the plurality of locations by using the determined transmission power.

As described above, the saturation of the PHY speed is suppressed by confirming the AGC value. Thus, according to the present example, underwater drone 30 can perform landing with higher accuracy.

Note that, in a case where it is confirmed that the PHY speed is saturated, underwater drone 30 may decrease the transmission power, retransmit the signal, and calculate or acquire an index value indicating a transmission path characteristic in the same manner as described above. In this case, the position in which the signal is first transmitted (the position in which it is confirmed that the PHY speed is saturated) may be the same as or different from the position in which the signal is retransmitted.

### [Third Alignment Example]

Aircraft parking apron 20 can also support wired communication by connecting wire (a control line or the like) to another aircraft parking apron 20, separately from the antenna. Further, it is also possible to realize a hybrid terminal, which utilizes wired and wireless communication in the above-described manner, in one communication scheme (for example, Large Scale Integration (LSI)).

In wireless power/data transmission network 1 in which such aircraft parking apron 20 is present, underwater drone 30 can determine the aircraft parking apron for landing and move toward the aircraft parking apron for landing based on topology information of the network. Thus, this alignment may be performed for multi-hop formation.

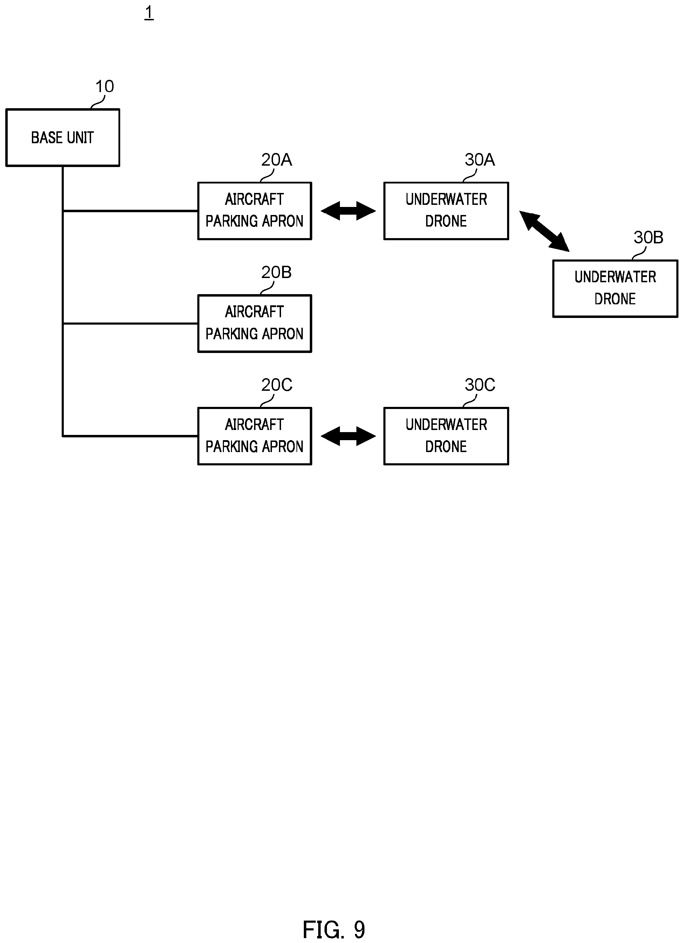

FIG. 9 illustrates a circumstance in which an aircraft parking apron for landing is determined based on topology information according to the present embodiment.

Wireless power/data transmission network 1 includes base unit 10, aircraft parking aprons 20A to 20C, and underwater drones 30A to 30C. Base unit 10 and aircraft parking aprons 20A to 20C are connected by wire, aircraft parking apron 20A and underwater drone 30A are connected wirelessly, and aircraft parking apron 20C and underwater drone 30C are connected wirelessly.

In this circumstance, it is assumed that underwater drone 30B has moved near underwater drone 30A, has performed authentication processing with base unit 10 via underwater drone 30A, and has participated in wireless power/data transmission network 1.

After participating in the network (after successful authentication) via the short-distance wireless processor, underwater drone 30B requests topology information (an example of the communication connection relationship according to the present disclosure) from base unit 10 (transmits a signal requesting topology information (also referred to as a topology information request)). Thereby, underwater drone 30B can receive (acquire) the topology information transmitted as a reply thereto from base unit 10. Alternatively, when aircraft parking apron 20A or underwater drone 30A holds topology information, aircraft parking apron 20A or underwater drone 30A may transmit the topology information to underwater drone 30B.

Topology information may include, for example, (the identification information (ID) of) base unit 10, (the ID of) aircraft parking apron 20, the connection relationship between base unit 10 and aircraft parking apron 20, the connection relationship between aircraft parking aprons 20, and the connection position of underwater drone 30 (for example, (the ID of) aircraft parking apron 20). Alternatively, topology information may be, for example, those described above from which the connection position of underwater drone 30 is excluded.

In the present embodiment and the other embodiment, the trigger to acquire topology information may be a case where a moving body such as underwater drone 30 determines that the moving body is located near (above in midair, directly below, or the like) the destination (external apparatus) based on position information utilizing GPS, or may be a case where the moving body determines that the moving body is located near (above in midair, directly below, or the like) the destination (external apparatus) based on camera images. Additionally or alternatively, the trigger to acquire topology information may be a case where a moving body such as underwater drone 30 receives or transmits a predetermined signal. The predetermined signal may be, for example, a signal for indicating the termination of the authentication processing described above or a signal requesting topology information. Further, the predetermined signal may be received or transmitted periodically (for example, once every few seconds, or the like), or may be started after the moving body determines that the moving body is located near (above in midair, directly below, or the like) the destination (external apparatus) based on position information utilizing GPS, camera images, and/or the like.

As described above, by acquiring topology information, underwater drone 30B can confirm that no other underwater drone (communication apparatus) is connected to aircraft parking apron 20B, and thus, underwater drone 30B can determine aircraft parking apron 20B as the aircraft parking apron for landing and move toward aircraft parking apron 20B based on camera images and/or the like.

Note that, in the present embodiment and the other embodiment, authentication processing may not be performed for underwater drone 30B to acquire topology information. For example, base unit 10, aircraft parking aprons 20A to 20C, and other aircraft parking aprons 30A and 30C may periodically transmit topology information like beacons, and underwater drone 30B may receive the topology information.

The underwater alignment example described above may be applied to a drone moving in midair (aerial drone) and to an aircraft parking apron present on land. **In** this case, when a desired aircraft parking apron is found, the aerial drone can move toward the aircraft parking apron based on position information utilizing GPS, camera images, and/or the like. Alternatively, the underwater alignment example described above may be applied to an aerial drone and a station having only a wireless data transmission function. Examples of such a station include a station for loading, unloading, or the like of a cargo.

### [Fourth Alignment Example]

An underwater drone is supposed to perform work (such as ocean exploration) in a work area in the sea (for example, on the seabed). In such a case, in order that the underwater drone transmits data or performs charging as appropriate, it is useful to connect a base unit with an aircraft parking apron or an underwater drone (which may also be referred to as a slave unit) in a multi-hop manner and cover the work area, in which the underwater drone performs work, in a planar manner to construct an IoT (Internet of Things) network (multi-hop network). When such a network is constructed (multi-hop formation is performed), it is desirable for the aircraft parking apron or the underwater drone to properly align with other communication apparatuses in order to cover a wide range of the work area.

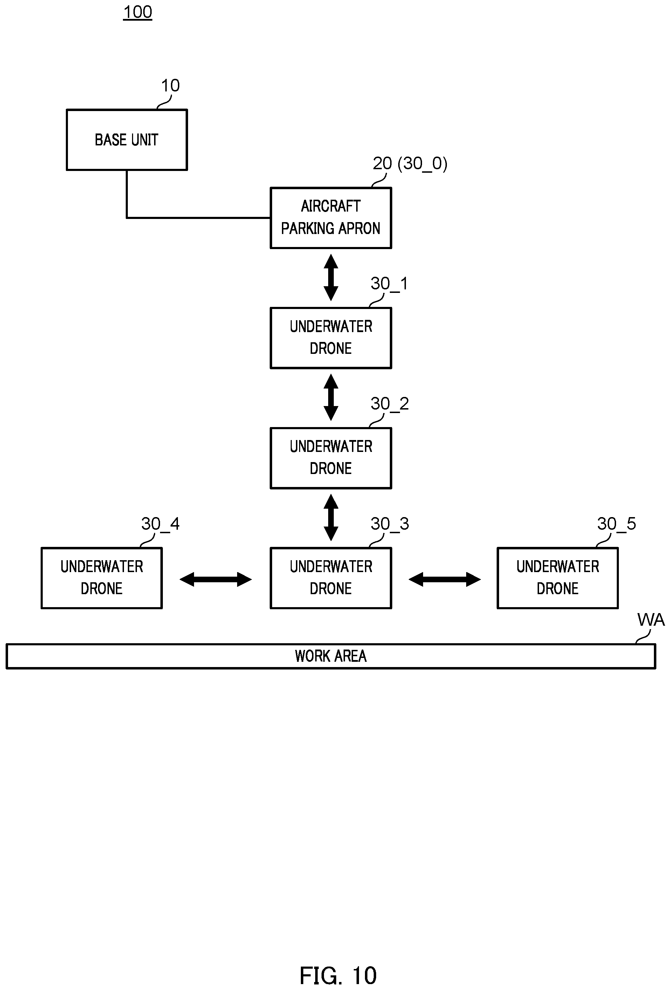

FIG. 10 illustrates an example of multi-hop network 100 according to the present embodiment.

Multi-hop network 100 has a multi-hop topology with five hops. In multi-hop network 100, aircraft parking apron 20 (realized by underwater drone 30_0, for example) is present below base unit 10, underwater drone 30_1 is present below (for example, directly below) underwater drone 30_0, underwater drone 30_2 is present below (for example, directly below) underwater drone 30_1, underwater drone 30_3 is present below (for example, directly below) underwater drone 30_2, and underwater drones 30_4 and 30_5 are deployed in the lateral direction along work area WA from underwater drone 30_3. With respect to work area WA, underwater drones may be deployed and disposed in the lateral direction in a planar manner.

In the fourth alignment example, an underwater drone first performs transmission path estimation while moving downward at the time of takeoff from an aircraft parking apron and measures the PHY speed, which is the opposite operation of landing at an aircraft parking apron. Note that, at this time, the underwater drone may perform transmission path estimation at a plurality of locations while moving in the horizontal direction, measure the PHY speeds, and determine whether the underwater drone has moved directly below. The arrangement is determined (a network is constructed) by a plurality of underwater drones treating a fixed underwater drone as an aircraft parking apron and repeating the same operation. Note that, the movement to a location roughly determined in advance may be performed by utilizing a camera.

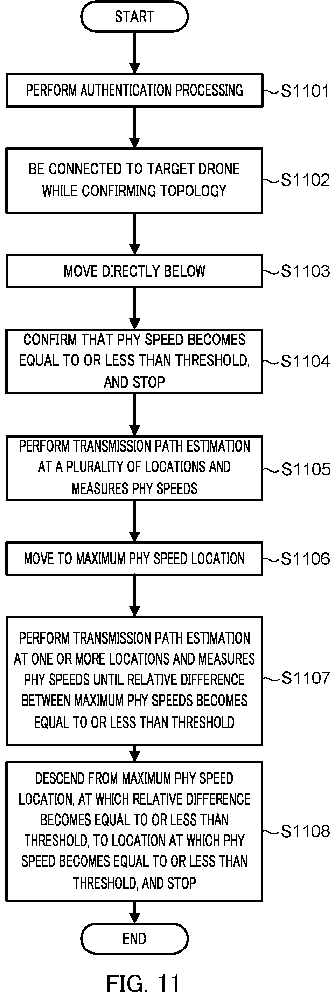

FIG. 11 is a flowchart illustrating an alignment example according to the present embodiment. This alignment may be performed, for example, for multi-hop formation. In this example, a topology in which underwater drones are connected in a vertical line is formed, and the underwater drone at an end of the vertical line (referred to as the target drone) is connected to the next underwater drone (referred to as the subject drone). Note that, it is assumed that a plurality of underwater drones has already been connected in a vertical line and a topology has been formed.

In step S1101, authentication processor 302 of the subject drone performs authentication processing with base unit 10 and participates in a network (for example, wireless power/data transmission network 1).

In step S1102, the subject drone, while confirming (by topology manager 304) a topology indicated by topology information acquired by transmitting a topology information request as appropriate (or by receiving topology information periodically) via the short-distance wireless processor, descends directly below the target drone under the control of movement controller 310 and is connected to the target drone.

In step S1103, the subject drone moves further directly below under the control of movement controller 310.

In step S1104, the subject drone performs transmission path estimation by measurer 306, confirms, while measuring the PHY speed, that the PHY speed becomes equal to or less than threshold α (for example, 40 Mbps), and stops under the control of movement controller 310.

In step S1105, the subject drone starts from the location at which the subject drone has stopped in step S1104, and moves each time by a predetermined distance in the horizontal direction under the control of movement controller 310. Thereby, the subject drone performs transmission path estimation at a predetermined number of a plurality of locations by measurer 306 and measures the PHY speeds. For example, the plurality of locations may be, albeit not limited to, four locations that are the vertices of an arbitrary square with the location, at which the subject drone has stopped in step S1104, as the center of gravity and with a diagonal twice the length of a predetermined distance.

In step S1106, under the control of movement controller 310, the subject drone moves to, among the plurality of locations, a location at which the PHY speed is the fastest (the maximum PHY speed location).

In step S1107, the subject drone starts from the location to which the subject drone has moved (at which the subject drone has stopped) in step S1106, and moves each time by a predetermined distance in the horizontal direction under the control of movement controller 310. Thereby, the subject drone performs transmission path estimation at one or more locations by measurer 306 and measures the PHY speeds until the relative difference (the absolute value of the difference) between the maximum PHY speed and the maximum PHY speed measured in step S1105 becomes equal to or less than threshold β (for example, 20 Mbps).

In step S1108, the subject drone performs transmission path estimation by measurer 306, and, under the control of movement controller 310, descends from the maximum PHY speed location, at which the relative difference becomes equal to or less than the threshold, to a location at which the PHY speed becomes equal to or less than threshold α, and stops.

Note that, the predetermined distance in steps S1105 and S1107 may be set so as to decrease stepwise according to the maximum PHY speed. For example, the predetermined distance may be initially set to a first value, such as 1 m, and in a case where the maximum PHY speed becomes equal to or greater threshold γ (for example, 80 Mbps), the predetermined distance may be set to a second value, such as 0.5 m, which is smaller than the first value.

In this way, the subject drone can move substantially directly below the target drone and enlarge the region covered by the network.

In this example, the network is enlarged in the vertical direction, but the network can also be enlarged in the lateral direction in the same manner.

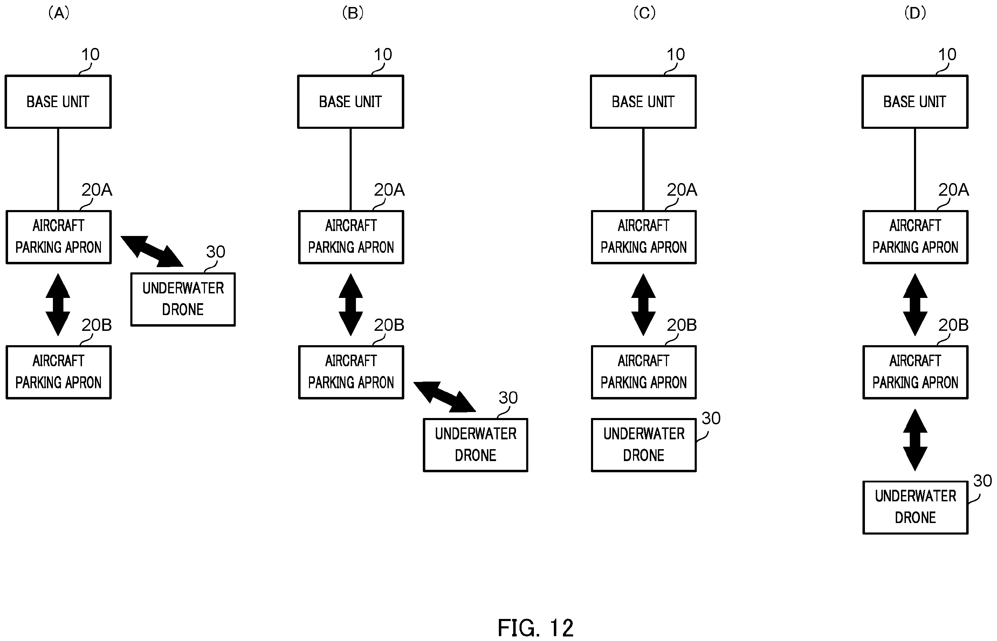

FIG. 12 illustrates that underwater drone 30, which is the subject drone, moves so as to be connected directly below aircraft parking apron 20B, which is the target drone, at an end in order to enlarge a network.

(A) and (B) of FIG. 12 correspond to steps S1102 and S1103 in FIG. 11, and illustrate that underwater drone 30 descends while confirming a topology and is connected aircraft parking apron 20A, which is changed such that underwater drone 30 is connected to aircraft parking apron 20B.

(B) of FIG. 12 also corresponds to step S1104 in FIG. 11, and illustrates that underwater drone 30 confirms that the PHY speed between underwater drone 30 and aircraft parking apron 20B becomes equal to or less than α, and stops.

(C) of FIG. 12 corresponds to steps S1105 to S1108 in FIG.11, and illustrates that underwater drone 30 is located directly below aircraft parking apron 20B.

(D) of FIG. 12 corresponds to step S1108 in FIG. 11, and illustrates that underwater drone 30 is somewhat away from aircraft parking apron 20B.

Hereinafter, some examples of multi-hop networks will be described.

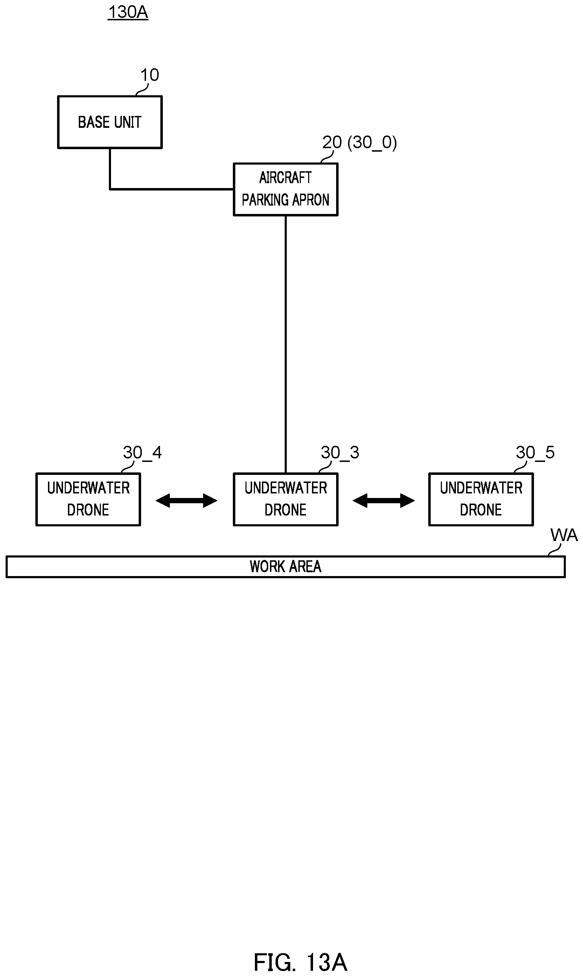

FIG. 13A illustrates an example of multi-hop network 130A having a multi-hop topology with three hops.

In multi-hop network 130A, aircraft parking apron 20 (realized by underwater drone 30_0, for example) is present below base unit 10, and underwater drone 30_3 is connected below (for example, directly below) underwater drone 30_0 by wire. The wire extends to the vicinity of work area WA. Underwater drones 30_4 and 30_5 are deployed in the lateral direction from underwater drone 30_3 along work area WA and are wirelessly connected to underwater drone 30_3. Note that, underwater drones 30_3, 30_4, and 30_5 may be connected by wire (all communication apparatuses may be connected by wire). Further, it is preferable for aircraft parking apron 20 to be located near the center of work area WA in order to cover the work area extensively with fewer hops.

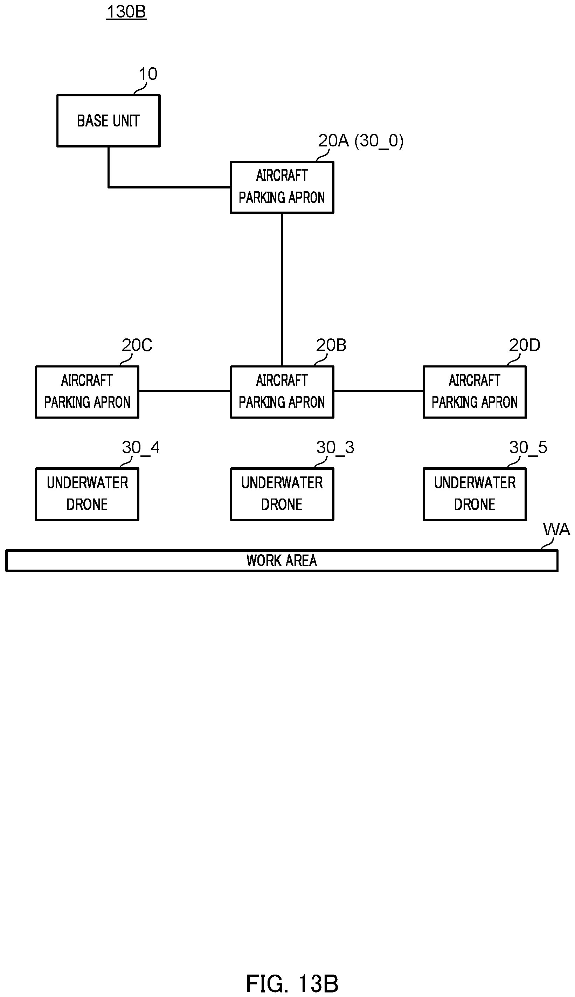

FIG. 13B illustrates an example of multi-hop network 130B having a multi-hop topology with two hops.

In multi-hop network 130B, aircraft parking apron 20A (realized by underwater drone 30_0, for example) is present below base unit 10, and aircraft parking apron 20B is connected below (for example, directly below) underwater drone 30_0 by wire. In addition, aircraft parking aprons 20C and 20D are deployed in the lateral direction from aircraft parking apron 20B along work area WA, and are connected to aircraft parking apron 20B by wire.

As described above, multi-hop network 130B takes a form in which the number of aircraft parking aprons is increased and underwater drones specific thereto land at aircraft parking aprons, respectively. For example, underwater drone 30_3 lands at aircraft parking apron 20B, underwater drone 30_4 lands at aircraft parking apron 20C, and underwater drone 30_4 lands at aircraft parking apron 20D. Note that, the connection between aircraft parking aprons may be a multi-hop wireless connection.

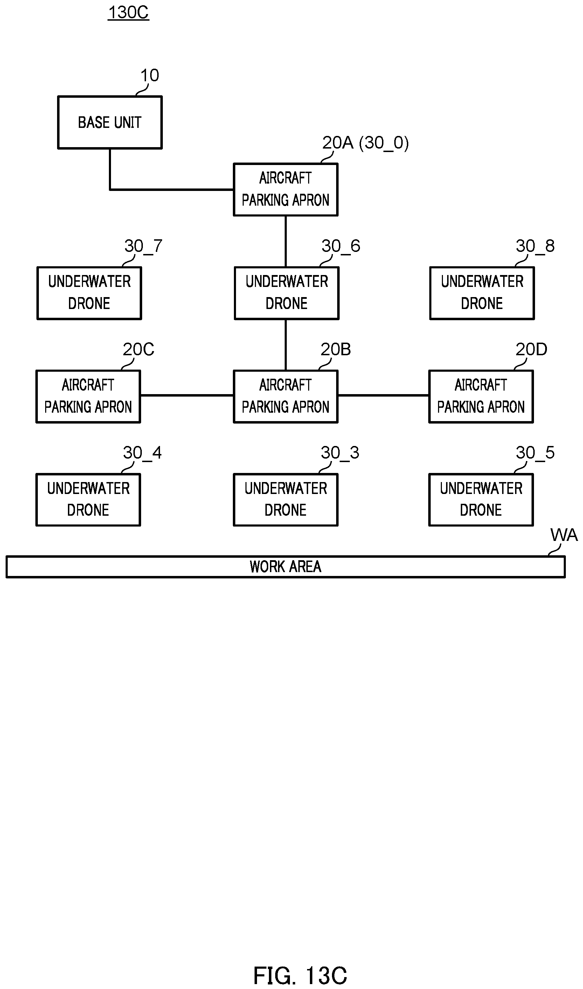

FIG. 13C illustrates an example of multi-hop network 130C having a multi-hop topology with two hops.

Multi-hop network 130C is the same as multi-hop network 120B, but differs from multi-hop network 130B in that multi-hop network 130C takes a form in which one or more underwater drones set in advance land at each aircraft parking apron. For example, underwater drone 30_3 or 30_6 lands at aircraft parking apron 20B, underwater drone 30_4 or 30_7 lands at aircraft parking apron 20C, and underwater drone 30_4 or 30_8 lands at aircraft parking apron 20D. The number of underwater drones that can land at an aircraft parking apron may be the same or different.

### [Operation Example]

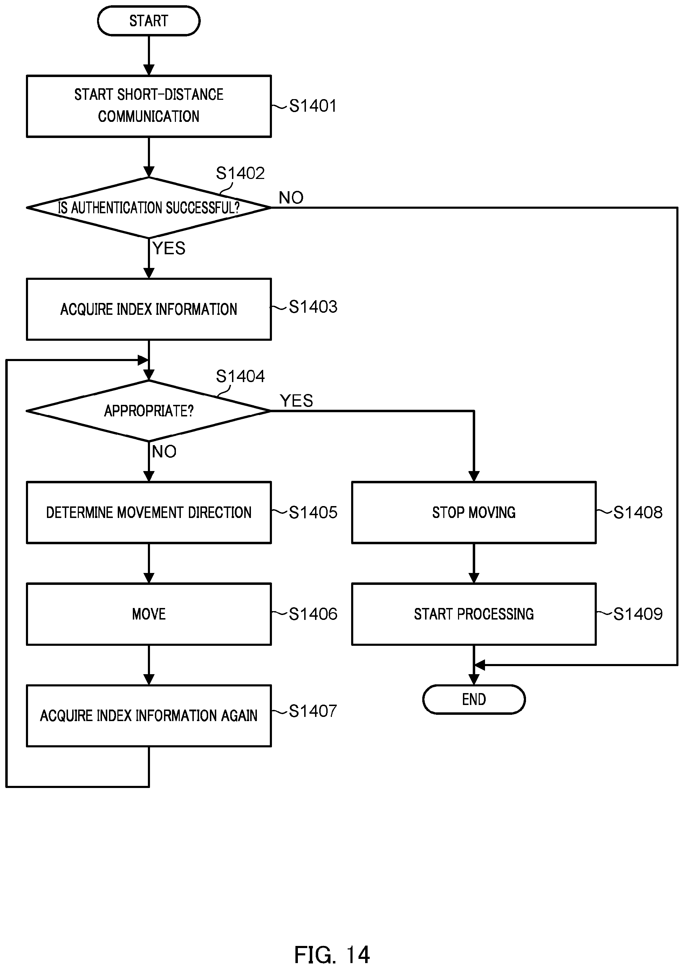

FIG. 14 is a flowchart illustrating an operation example of underwater drone 30 according to the present embodiment.

In step S1401, underwater drone 30 starts, via the short-distance wireless processor, short-distance communication with another communication apparatus that is participating in wireless power/data transmission network 1 and is present at a short distance.

In step S1402, authentication processor 302 of underwater drone 30 performs authentication processing with base unit 10 to determine whether authentication is successful.

In a case where the authentication is unsuccessful (NO in step S1402), the flow ends.

In a case where the authentication is successful (YES in step S1402), on the other hand, topology manager 304 or measurer 306 (referred to as the index acquirer or the index calculator) of underwater drone 30 acquires (calculates) index information in step S1403. The index information may be, for example, (an index value indicating) a transmission path characteristic (the SN ratio, the tone map, the PHY speed, or the like) between underwater drone 30 and the other communication apparatus, or may be a communication connection relationship (topology information or the like) in wireless power/data transmission network 1.

In step S1404, movement controller 310 of underwater drone 30 determines based on the index information acquired in step S1403 or the like whether the current location is appropriate.

In a case where the current location is appropriate (YES in step S1404), underwater drone 30 stops moving under the control of movement controller 310 in step S1408.

In step S1409, underwater drone 30 starts processing such as performing work, transmitting and receiving data, or supplying power or receiving power. The flow then ends.

In a case where the current location is not appropriate (NO in step S1404), on the other hand, movement controller 310 of underwater drone 30 determines the movement direction in step S1405.

In step S1406, underwater drone 30 moves, under movement controller 310, in the movement direction determined in step S1405.

In step S1407, the index acquirer of underwater drone 30 acquires the index information again. Thereafter, the flow returns to step S1404 and the above-described processing is repeated.

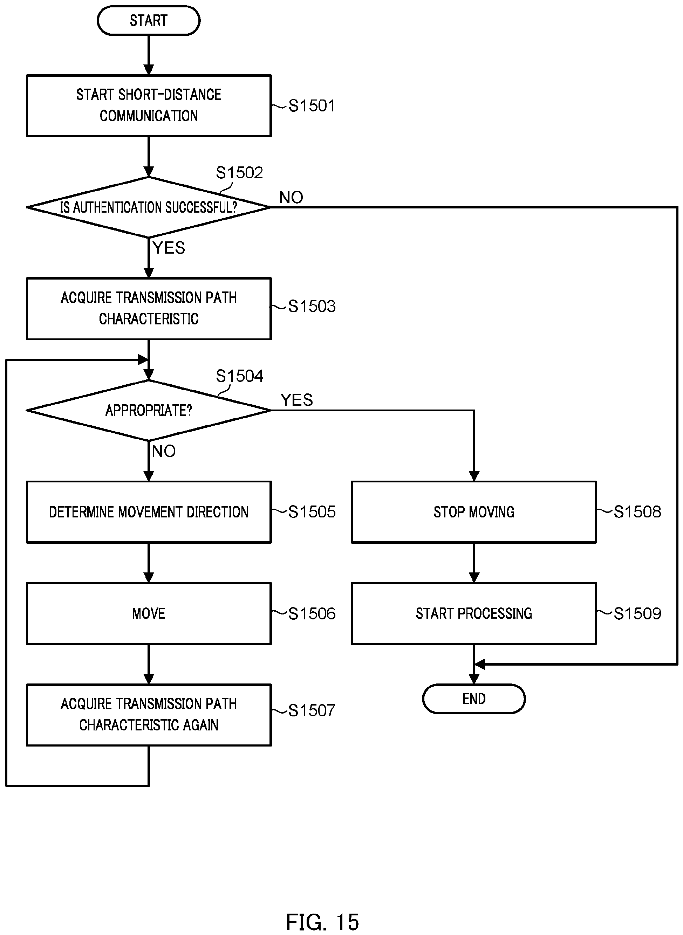

FIG. 15 is a flowchart illustrating an operation example of underwater drone 30 according to the present embodiment.

In step S1501, underwater drone 30 starts, via the short-distance wireless processor, short-distance communication with another communication apparatus that is participating in wireless power/data transmission network 1 and is present at a short distance.

In step S1502, authentication processor 302 of underwater drone 30 performs authentication processing with base unit 10 to determine whether authentication is successful.

In a case where the authentication is unsuccessful (NO in step S1502), the flow ends.

In a case where the authentication is successful (YES in step S1502), on the other hand, measurer 306 of underwater drone 30 acquires a transmission path characteristic at one or more locations in step S1503. The transmission path characteristic may be, for example, the SN ratio, the tone map, the PHY speed, or the like. Note that, in a case where underwater drone 30 moves in the horizontal direction and acquires a transmission path characteristic at a plurality of locations, underwater drone 30 moves (for example, slowly), regardless of the calculated transmission path characteristic, to another location in a direction determined in advance, in a randomly determined direction, or in a direction determined based on camera images, under the control of movement controller 310.

In step S1504, movement controller 310 of underwater drone 30 determines based on the transmission path characteristic acquired in step S1503 whether the current location is appropriate.

In a case where the current location is appropriate (YES in step S1504), underwater drone 30 stops moving under the control of movement controller 310 in step S1508.

For example, in a case where underwater drone 30 performs alignment for charging and/or data communication, movement controller 310 determines, when the transmission path characteristic (PHY speed) at the current location is equal to or greater than a threshold, that the current location is appropriate, and causes underwater drone 30 to stop at the current location.

Further, for example, in a case where underwater drone 30 performs alignment for multi-hop formation, movement controller 310 determines, when the transmission path characteristic (PHY speed) at the current location is equal to or less than a threshold, that the current location is appropriate, and causes underwater drone 30 to stop at the current location.

In step S1509, underwater drone 30 starts processing such as performing work, transmitting and receiving data or performing charging. The flow then ends.

In a case where the current location is not appropriate (NO in step S1504), on the other hand, movement controller 310 of underwater drone 30 determines the movement direction in step S1505.

For example, in a case where underwater drone 30 performs alignment for charging and/or data communication, movement controller 310 determines, when the current location is not the location at which, among the transmission path characteristics (PHY speeds) measured at a plurality of locations, the maximum transmission path characteristic is measured, that the current location is not appropriate, and determines that the movement direction is the horizontal direction and is a direction toward the location at which the maximum transmission path characteristic is measured (a direction in which the transmission path characteristic increases).

Further, for example, in a case where underwater drone 30 performs alignment for charging and/or data communication, movement controller 310 determines, when the transmission path characteristic (PHY speed) at the current location is less than a threshold, that the current location is not appropriate, and determines that the movement direction is the horizontal direction and is a direction determined in advance, a randomly determined direction, or a direction determined based on camera images.

Further, for example, in a case where underwater drone 30 performs alignment for multi-hop formation, movement controller 310 determines, when the transmission path characteristic (PHY speed) at the current location is greater than a threshold, that the current location is not appropriate, and determines that the movement direction is a directly-below direction (a direction in which the transmission path characteristic decreases) which is a direction away from the external apparatus that is the counterpart with which the transmission path characteristic has been acquired.

In step S1506, underwater drone 30 moves, under the control of movement controller 310, in the movement direction determined in step S1505.

In step S1507, measurer 306 of underwater drone 30 acquires the transmission path characteristic again. Thereafter, the flow returns to step S1504 and the above-described processing is repeated.

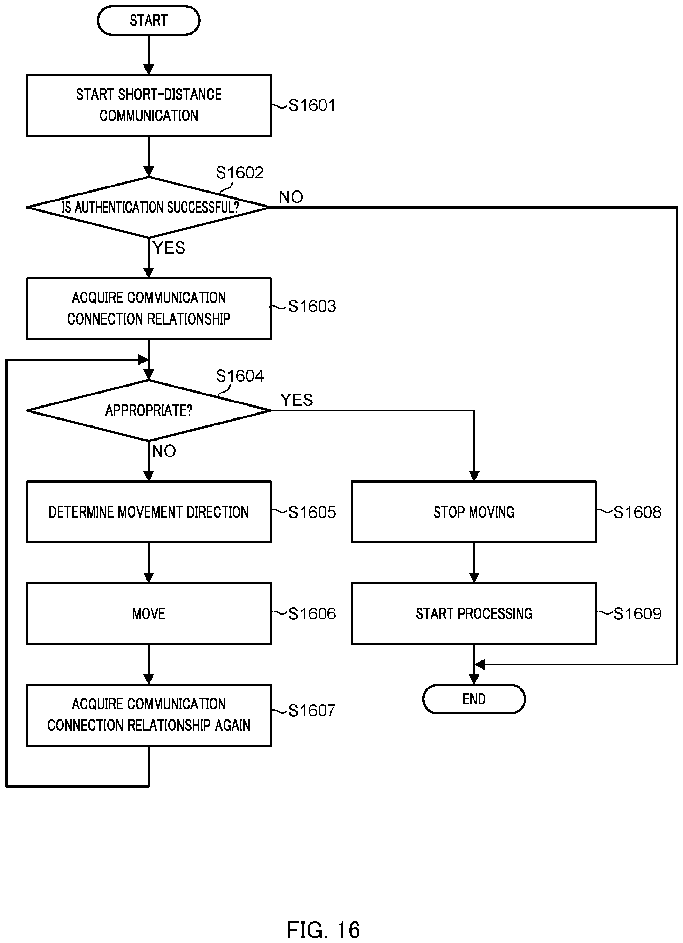

FIG. 16 is a flowchart illustrating an operation example of underwater drone 30 according to the present embodiment.

In step S1601, underwater drone 30 starts, via the short-distance wireless processor, communication with another communication apparatus that is participating in wireless power/data transmission network 1 and is present at a short distance.

In step S1602, authentication processor 302 of underwater drone 30 performs authentication processing with base unit 10 to determine whether authentication is successful.

In a case where the authentication is unsuccessful (NO in step S1602), the flow ends.

In a case where the authentication is successful (YES in step S1602), on the other hand, topology manager 304 of underwater drone 30 acquires a communication connection relationship of wireless power/data transmission network 1 in step S1603. The communication connection relationship may be, for example, topology information of wireless power/data transmission network 1.

In step S1604, movement controller 310 of underwater drone 30 determines, based on the communication connection relationship acquired in step S1603, whether the current location is appropriate.

In a case where the current location is appropriate (YES in step S1604), underwater drone 30 stops moving under the control of movement controller 310 in step S1608.

For example, in a case where underwater drone 30 performs alignment for forming a desired topology, movement controller 310 of underwater drone 30 determines, when the current location is the desired connection position (for example, an empty aircraft parking apron) based on topology information, that the current location is appropriate, and causes underwater drone 30 to stop at the current location.

In step S1609, underwater drone 30 starts processing such as performing work, transmitting and receiving data or performing charging. The flow then ends.

In a case where the current location is not appropriate (NO in step S1604), on the other hand, movement controller 310 of underwater drone 30 determines the movement direction in step S1605.

For example, in a case where underwater drone 30 performs alignment for forming a desired topology, movement controller 310 of underwater drone 30 determines, when the current location is not the desired connection position (for example, an empty aircraft parking apron) based on topology information, that the current location is not appropriate, and determines that the movement direction is a direction toward the destination, such as another aircraft parking apron or an underwater drone.

In step S1606, underwater drone 30 moves, under the control of movement controller 310, in the movement direction determined in step S1605.

For example, underwater drone 30 moves, based on camera images and/or the like, in the direction to the destination, under the control of movement controller 310. Note that, in the case of an aerial drone, the aerial drone may move, based on position information utilizing GPS or the like as well, in the direction to the destination under the control of movement controller 310.

In step S1607, topology manager 304 of underwater drone 30 acquires the communication connection relationship again. Thereafter, the flow returns to step S1604 and the above-described processing is repeated.

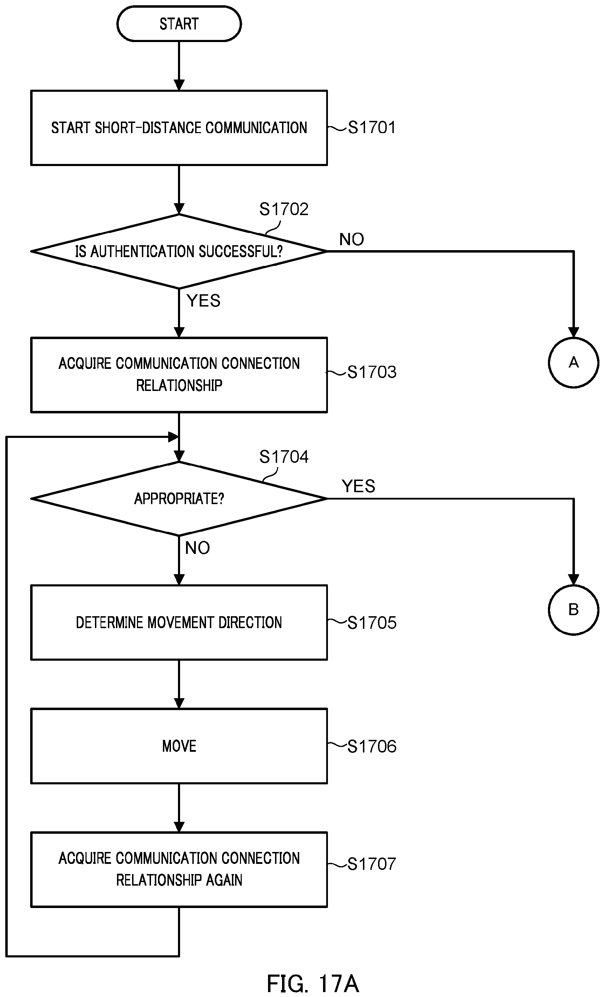

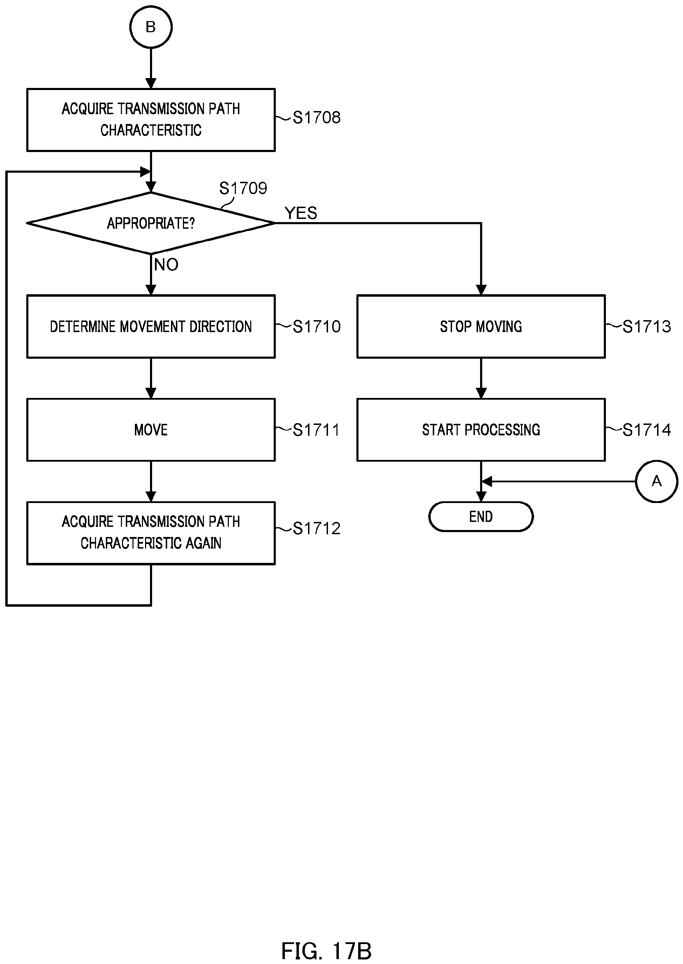

FIGS. 17A and 17B are flowcharts illustrating an operation example of underwater drone 30 according to the present embodiment.

In step S1701, underwater drone 30 starts, via the short-distance wireless processor, communication with another communication apparatus that is participating in wireless power/data transmission network 1 and is present at a short distance.

In step S1702, authentication processor 302 of underwater drone 30 performs authentication processing with base unit 10 to determine whether authentication is successful.

In a case where the authentication is unsuccessful (NO in step S1702), the flow ends.

In a case where the authentication is successful (YES in step S1702), on the other hand, topology manager 304 of underwater drone 30 acquires a communication connection relationship of wireless power/data transmission network 1 in step S1703. The communication connection relationship may be, for example, topology information of wireless power/data transmission network 1.

In step S1704, movement controller 310 of underwater drone 30 determines, based on the communication connection relationship acquired in step S1703, whether the current location is appropriate.

In a case where the current location is appropriate (YES in step S1704), the flow proceeds to step S1708.

For example, in a case where underwater drone 30 performs alignment for forming a desired topology, movement controller 310 determines, when the current location is the desired connection position (for example, an end of a topology) based on topology information, that the current location is appropriate.

In a case where the current location is not appropriate (NO in step S1704), movement controller 310 of underwater drone 30 determines the movement direction in step S1705.

For example, in a case where underwater drone 30 performs alignment for forming a desired topology, movement controller 310 determines, when the current location is not the desired connection position (for example, an end of a topology) based on topology information, that the current location is not appropriate, and determines that the movement direction is a direction along the movement route (for example, the direction of a communication apparatus present at an end (for example, directly below)).

In step S1706, underwater drone 30 moves, under the control of movement controller 310, in the movement direction determined in step S1705.

In step S1707, topology manager 304 of underwater drone 30 acquires the communication connection relationship again. Thereafter, the flow returns to step S1704 and the above-described processing is repeated.

In step S1708, measurer 306 of underwater drone 30 acquires a transmission path characteristic at one or more locations. The transmission path characteristic may be, for example, the SN ratio, the tone map, the PHY speed, or the like. Note that, in a case where underwater drone 30 moves in the horizontal direction and acquires a transmission path characteristic at a plurality of locations, underwater drone 30 moves (for example, slowly), regardless of the calculated transmission path characteristic, to another location in a direction determined in advance, a randomly determined direction, or a direction determined based on camera images, under the control of movement controller 310.

In step S1709, movement controller 310 of underwater drone 30 determines based on the transmission path characteristic acquired in step S1708 whether the current location is appropriate.

In a case where the current location is appropriate (YES in step S1709), underwater drone 30 stops moving under the control of movement controller 310 in step S1713.

For example, in a case where underwater drone 30 performs alignment for forming a desired topology, movement controller 310 determines, when the transmission path characteristic (PHY speed) at the current location is equal to or less than a threshold, that the current location is appropriate, and causes underwater drone 30 to stop at the current location.

In step S1714, underwater drone 30 starts processing such as performing work, transmitting and receiving data or performing charging. The flow then ends.

In a case where the current location is not appropriate (NO in step S1709), on the other hand, movement controller 310 of underwater drone 30 determines the movement direction in step S1710.

For example, in a case where underwater drone 30 performs alignment for forming a desired topology, movement controller 310 determines, when the transmission path characteristic (PHY speed) at the current location is greater than a threshold, that the current location is not appropriate, and determines that the movement direction is a directly-below direction (a direction in which the transmission path characteristic decreases) which is a direction away from the external apparatus that is the counterpart with which the transmission path characteristic has been acquired.

In step S1711, underwater drone 30 moves, under the control of movement controller 310, in the movement direction determined in step S1710.

In step S1712, measurer 306 of underwater drone 30 acquires the transmission path characteristic again. Thereafter, the flow returns to step S1709 and the above-described processing is repeated.

The functional processor of CPU 201 described in the present embodiment may be integrated with another functional processor or may be divided into two or more sub-functional processors as appropriate. Further, the order of steps illustrated in flowcharts or the like described in the present embodiment is not limited to the illustrated order.

### <Effects in Embodiment 1>

Underwater drone 30 according to the present embodiment performs wireless communication with an external apparatus such as base unit 10, aircraft parking apron 20, and another underwater drone 30 via short-distance antenna 210 and short-distance wireless communication circuitry 211. CPU 201 (measurer 306) of underwater drone 30 acquires an index value indicating a transmission path characteristic (the SN ratio, the tone map, the PHY speed, or the like) between underwater drone 30 and the external apparatus by transmitting and receiving signals to and from the external apparatus. CPU 201 (movement controller 310) of underwater drone 30 determines, based on the index value, the position or the direction to or in which underwater drone 30 moves, and causes underwater drone 30 to move to the determined position or in the determined direction. Thus, the position or the direction to or in which underwater drone 30 moves is determined based on the index value indicating the transmission path characteristic between underwater drone 30 and the external apparatus, which is less susceptible to environmental factors around underwater drone 30, and underwater drone 30 is controlled such that underwater drone 30 moves to the determined position or in the determined direction, and thus, it is possible to improve the alignment accuracy.

In addition, underwater drone 30 according to the present embodiment performs wireless communication with an external apparatus such as base unit 10, aircraft parking apron 20 and another underwater drone 30 via short-distance antenna 210 and short-distance wireless communication circuitry 211. CPU 201 (topology manager 304) of underwater drone 30 acquires topology information of a communication network (for example, wireless power/data transmission network 1) from base unit 10 or the like, by transmitting a topology information request, or periodically without transmitting a topology information request. CPU 201 (movement controller 310) of underwater drone 30 determines, based on the topology information, the position or the direction to or in which underwater drone 30 moves, and causes underwater drone 30 to move to the determined position or in the determined direction. Thus, the position or the direction to or in which underwater drone 30 moves is determined based on the topology information of the communication network, which is less susceptible to environmental factors around underwater drone 30, and underwater drone 30 is controlled such that underwater drone 30 moves to the determined position or in the determined direction, and thus, it is possible to improve the alignment accuracy.

### <Summary of Embodiment 1>

A moving body according to an exemplary embodiment of the present disclosure includes: a communicator that performs wireless communication with an external apparatus; an index acquirer that acquires a first index value indicating a transmission path characteristic between the moving body and the external apparatus; and a movement controller that determines, based on the first index value, a first position or a first direction to or in which the moving body moves, and causes the moving body to move to the first position or in the first direction.

**In** the present moving body, the index acquirer acquires a plurality of the first index values corresponding to a plurality of locations, respectively, and the movement controller determines that the first position is, among the plurality of locations, a location at which a maximum value of the plurality of first index values is acquired.

**In** the present moving body, the index acquirer acquires, based on a first signal transmitted by the communicator with first transmission power to the external apparatus, the first index value, the index acquirer acquires, based on a second signal transmitted by the communicator with second transmission power lower than the first transmission power to the external apparatus, a second index value indicating the transmission path characteristic, after the moving body moves to the first position, and the movement controller determines, based on the second index value, a second position or a second direction to or in which the moving body moves, and causes the moving body to move to the second position or in the second direction.

**In** the present moving body, the movement controller determines that the first direction is a direction away from the external apparatus in a case where the first index value is higher than a first threshold.

**In** the present moving body, the movement controller causes the moving body to stop at a location at which the first index value is acquired, in a case where the first index value is equal to or less than the first threshold.

A movement control method according to an exemplary embodiment of the present disclosure includes: acquiring, by a moving body that performs wireless communication with an external apparatus, a transmission path characteristic between the moving body and the external apparatus; determining, by the moving body, a position or a direction to or in which the moving body moves, based on the index value; and causing, by the moving body, the moving body to move to the position or in the direction.

A moving body according to an exemplary embodiment of the present disclosure includes: a communicator that wirelessly performs communication with an external apparatus; an acquirer that acquires a communication connection relationship of a communication network including the external apparatus and another communication apparatus, by the communication with the external apparatus; and a movement controller that determines, based on the communication connection relationship, a first direction, in which the moving body moves, and causes the moving body to move in the first direction.

In the present moving body, in a case where the communication connection relationship indicates that the other communication apparatus is present at an end of the communication network, the movement controller determines that the first direction is a direction of the other communication apparatus.

The present moving body further includes an index acquirer that acquires an index value indicating a transmission path characteristic between the moving body and the other external apparatus, and the movement controller determines, based on the index value, a second direction, in which the moving body moves, and causes the moving body to move in the second direction.

In the present moving body, in a case where the index value is higher than a threshold, the movement controller determines that the second direction is a direction away from the other communication apparatus.

In the present moving body, in a case where the communication connection relationship indicates that no communication apparatus wirelessly connected to the other communication apparatus is present, the movement controller determines that the first direction is a direction of the other communication apparatus.

A movement control method according to an exemplary embodiment of the present disclosure includes: acquiring, by a moving body that performs communication with an external apparatus wirelessly, a communication connection relationship of a communication network including the external apparatus and another communication apparatus, by the communication with the external apparatus; determining, by the moving body, a direction in which the moving body moves, based on the communication connection relationship; and causing, by the moving body, the moving body to move in the direction.

### (Embodiment 2)

### <Configuration of Wireless Power/Data Transmission System>

In Embodiment 1, the wireless power/data transmission system is mainly disposed on the water (for example, on the sea) and underwater (for example, in the sea) has been described, whereas in Embodiment 2, the wireless power/data transmission system may be disposed on the sea and in the sea, may be disposed on land and in midair, may be disposed on fresh water and in fresh water, or the disposition may be a combination thereof. Wireless power/data transmission system 1 according to Embodiment 1 includes underwater drone 30, whereas in the wireless power/data transmission system according to Embodiment 2, underwater drone 30 is read as drone 30' encompassing an underwater drone and an aerial drone (or a drone capable of moving underwater, in midair, and on land (hereinafter referred to as an amphibious drone)). Drone 30' is an example of the "moving body" according to the present disclosure.

### <Configuration of Wireless Power/Data Transmission System>

### [Configuration of Drone]

FIG. 18 illustrates an exemplary functional configuration of CPU 201 of drone 30'. Note that, descriptions of the same elements as those of drone 30 will be omitted.

As illustrated in FIG. 18, drone 30' includes user input information acquirer 311, sensor information acquirer 312, mode determiner 313, and profile selector 314 in addition to the elements illustrated in FIG. 3.

User input information acquirer 311 acquires, from the user, information (user input information) inputted by the user via UI 203 or via a mobile communication network and a browser or the like. The user input information may be information for designating the mode related to the operating environment of drone 30' (the mode in which drone 30' operations), for example. Examples of such modes may include a midair mode, a sea water mode, and a fresh water mode, or may include a midair mode and an underwater mode.

Sensor information acquirer 312 acquires, from sensor 207, sensor information measured or sensed by sensor 207. The sensor information includes, for example, information indicating whether moisture has been detected (or is present), the salinity, the atmospheric pressure, and/or the like. Note that, the sensor information may include position information utilizing GPS. That is, GPS receiver 205 may be interpreted as a part of sensor 207.

Mode determiner 313 determines the mode related to the operating environment of drone 30' (such as the medium in which drone 30' moves, the medium in which drone 30' is present, or the like) based on the user input information, the sensor information, and/or the like.

Profile selector 314 selects the PHY speed profile, which corresponds to the mode determined by mode determiner 313. Profile selector 314 is an example of the "selector" according to the present disclosure.

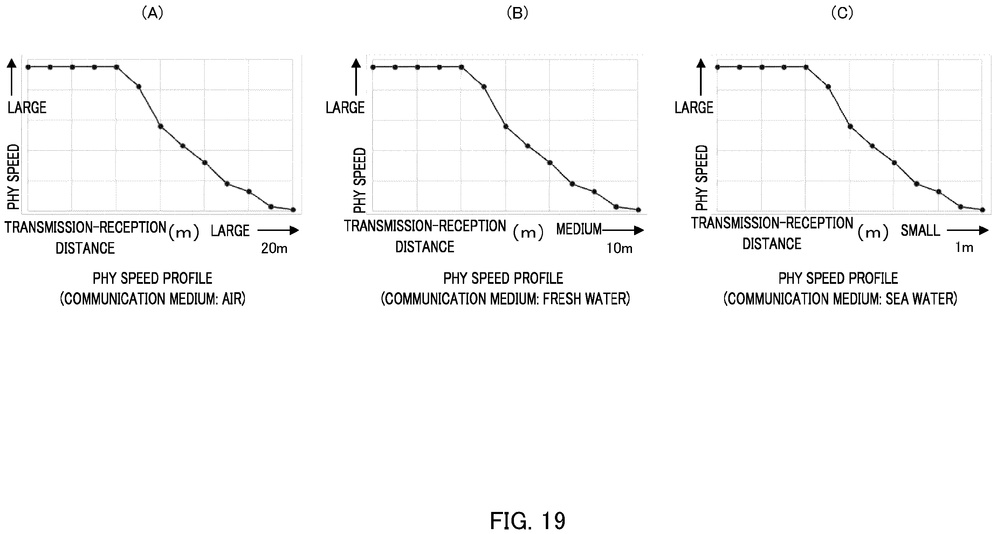

FIG 19 illustrates PHY speed profiles in different communication media in the case of the same and a constant transmission power.

(A) in FIG. 19 illustrates the PHY speed profile in a case where the communication medium is air, (B) in FIG. 19 illustrates the PHY speed profile in a case where the communication medium is fresh water, and (C) in FIG. 19 illustrates the PHY speed profile in a case where the communication medium is sea water. Note that, in a case where drone 30' is an amphibious drone, drone 30' may hold a profile for midair as illustrated in (A) of FIG. 19, a profile for fresh water as illustrated in (B) of FIG. 19, and a profile for sea water as illustrated in (C) of FIG. 19, in memory 202. Further, in a case where drone 30' is an underwater drone, drone 30' may hold a profile for fresh water as illustrated in (B) of FIG. 19 and a profile for sea water as illustrated in (C) of FIG. 19, in memory 202.

As illustrated in FIG. 19, in a case where the communication medium is air, the transmission-reception distance (communication distance) is the longest; in a case where the communication medium is fresh water, the transmission-reception distance is shorter than that in a case where the communication medium is air; and in a case where the communication medium is sea water, the transmission-reception distance is much shorter.

As described above, in the case of different communication media, the transmission-reception distances differ even with the same PHY speed, and thus, it is impossible to estimate the distance appropriately unless an appropriate profile is selected according to the environment (that is, the communication medium) in which the drone moves.

In view of the above, a solution for estimating the distance between drone 30' and the communication counterpart appropriately even when drone 30' moves through different communication media will be described.

### <Operation of Wireless Power/Data Transmission System>

### [First Mode Determination Example]

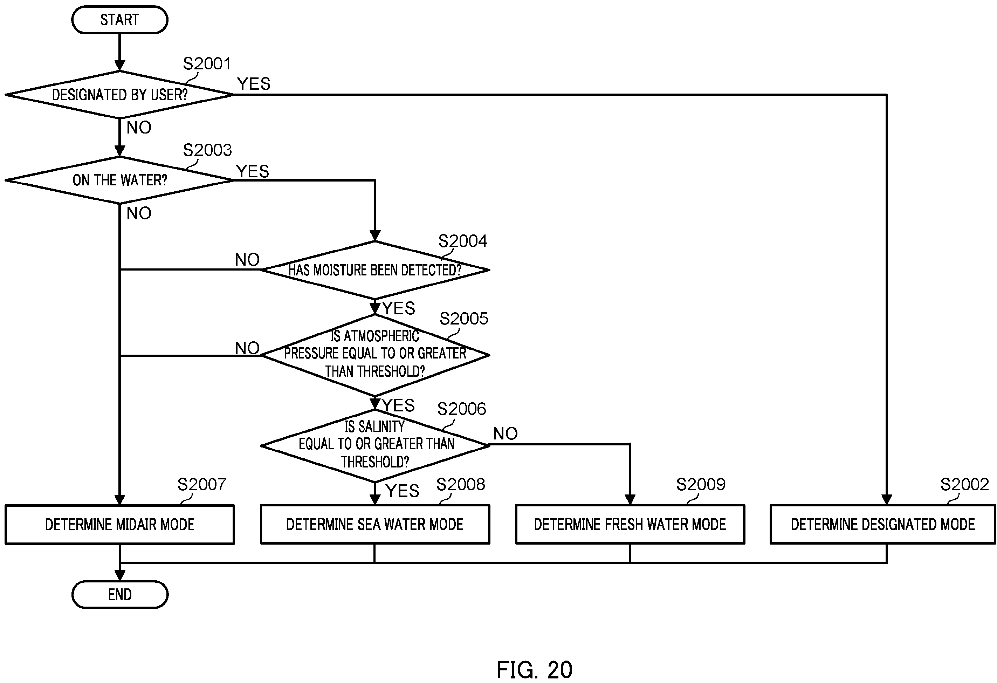

FIG. 20 is a flowchart illustrating a mode determination example according to the present embodiment.

In step S2001, mode determiner 313 determines, based on the user input information acquired by user input information acquirer 311, whether the mode has been designated by the user.

In a case where the mode has been designated by the user (YES in step S2001), mode determiner 313 determines in step S2002 that the mode related to the operating environment of drone 30' is the mode designated by the user. The flow then ends.

In a case where the mode has not been designated by the user (NO in step S2001), on the other hand, mode determiner 313 determines in step S2003, by using information on a position (for example, the latitude and longitude) measured by GPS receiver 205 and map information saved in memory 202, whether drone 30' is present on the water. Here, the term: on the water does not mean that drone 30' is in contact with water, but rather means that the measured latitude and longitude overlap with the sea, a lake, or the like.

In a case where drone 30' is not present on the water (NO in step S2003), mode determiner 313 determines in step S2007 that the mode related to the operating environment of drone 30' is the midair mode. The flow then ends.

In a case where drone 30' is present on the water (YES in step S2003), on the other hand, mode determiner 313 determines in step S2004, based on the sensor information acquired by sensor information acquirer 312, whether moisture has been detected.

In a case where no moisture has been detected (NO in step S2004), mode determiner 313 determines in step S2007 that the mode related to the operating environment of drone 30' is the midair mode. The flow then ends.

In a case where moisture has been detected (YES in step S2004), on the other hand, mode determiner 313 determines in step S2005, based on the sensor information acquired by sensor information acquirer 312, whether the atmospheric pressure is equal to or greater than a threshold.

In a case where the atmospheric pressure is not equal to or greater than the threshold (NO in step S2005), mode determiner 313 determines in step S2007 that the mode related to the operating environment of drone 30' is the midair mode. The flow then ends.

In a case where the atmospheric pressure is equal to or greater than the threshold (YES in step S2005), on the other hand, mode determiner 313 determines in step S2006, based on the sensor information acquired by sensor information acquirer 312, whether the salinity is equal to or greater than a threshold.

In a case where the salinity is equal to or greater than the threshold (YES in step S2006), mode determiner 313 determines in step S2008 that the mode related to the operating environment of drone 30' is the sea water mode. The flow then ends.

In a case where the salinity is not equal to or greater than the threshold (NO in step S2006), on the other hand, mode determiner 313 determines in step S2009 that the mode related to the operating environment of drone 30' is the fresh water mode. The flow then ends.

Note that, in a case where drone 30' is not an amphibious drone in the first mode determination example, step S2003 may be skipped.

### [Second Mode Determination Example]

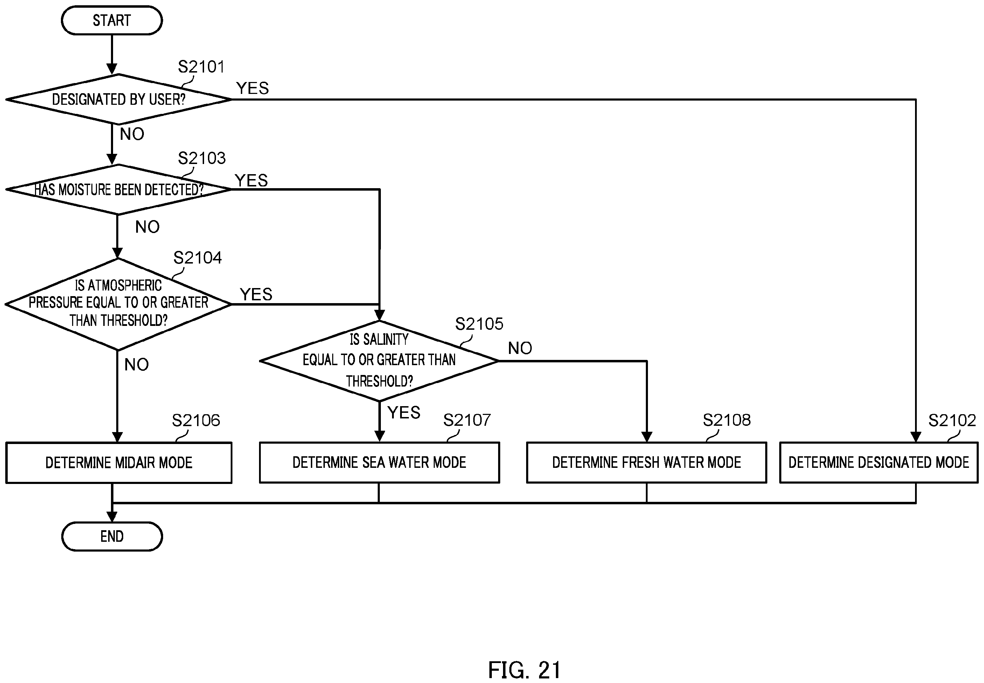

FIG. 21 is a flowchart illustrating a mode determination example according to the present embodiment. In this example, it is supposed that drone 30' does not utilize or have the GPS function.

In step S2101, mode determiner 313 determines, based on the user input information acquired by user input information acquirer 311, whether the mode has been designated by the user.

In a case where the mode has been designated by the user (YES in step S2101), mode determiner 313 determines in step S2102 that the mode related to the operating environment of drone 30' is the mode designated by the user. The flow then ends.

In a case where the mode has not been designated by the user (NO in step S2101), on the other hand, mode determiner 313 determines in step S2103, based on the sensor information acquired by sensor information acquirer 312, whether moisture has been detected.

In a case where no moisture has been detected (NO in step S2103), mode determiner 313 determines in step S2104, based on the sensor information acquired by sensor information acquirer 312, whether the atmospheric pressure is equal to or greater than a threshold.

In a case where moisture has been detected (YES in step S2103), on the other hand, the flow proceeds to step S2105.

In a case where the atmospheric pressure is not equal to or greater than the threshold (NO in step S2104), mode determiner 313 determines in step S2106 that the mode related to the operating environment of drone 30' is the midair mode. The flow then ends.

In a case where the atmospheric pressure is equal to or greater than the threshold, on the other hand (YES in step S2104), the flow proceeds to step S2105.

In step S2105, mode determiner 313 determines, based on the sensor information acquired by sensor information acquirer 312, whether the salinity is equal to or greater than a threshold.

In a case where the salinity is equal to or greater than the threshold (YES in step S2105), mode determiner 313 determines in step S2107 that the mode related to the operating environment of drone 30' is the sea water mode. The flow then ends.

In a case where the salinity is not equal to or greater than the threshold (NO in step S2105), on the other hand, mode determiner 313 determines in step S2108 that the mode related to the operating environment of drone 30' is the fresh water mode. The flow then ends.

Note that, in a case where drone 30' is not an amphibious drone in the second mode determination example, steps S2103 and S2104 may be skipped.

### [Third Mode Determination Example]

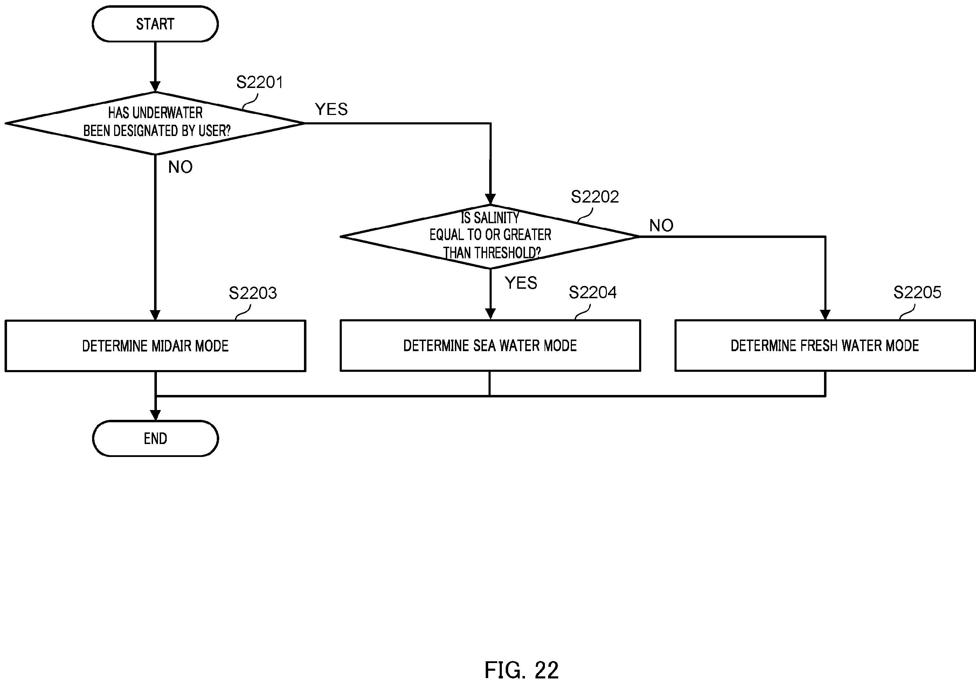

FIG. 22 is a flowchart illustrating a mode determination example according to the present embodiment. In this example, the user designates whether drone 30' operates in the midair mode or in the underwater mode, and in a case where drone 30' operates in the underwater mode, it is automatically determined whether drone 30' operates in the fresh water mode or in the sea water mode.

In step S2201, mode determiner 313 determines, based on the user input information acquired by user input information acquirer 311, whether the underwater mode has been designated by the user.

In a case where the underwater mode has not been designated by the user (NO in step S2201), mode determiner 313 determines in step S2203 that the mode related to the operating environment of drone 30' is the midair mode. The flow then ends.

In a case where the underwater mode has been designated by the user (YES in step S2201), on the other hand, mode determiner 313 determines in step S2202, based on the sensor information acquired by sensor information acquirer 312, whether the salinity is equal to or greater than a threshold.

In a case where the salinity is equal to or greater than the threshold (YES in step S2202), mode determiner 313 determines in step S2204 that the mode related to the operating environment of drone 30' is the sea water mode. The flow then ends.

In a case where the salinity is not equal to or greater than the threshold (NO in step S2202), on the other hand, mode determiner 313 determines in step S2205 that the mode related to the operating environment of drone 30' is the fresh water mode. The flow then ends.

In the above-described first to third mode determination examples, the midair mode may be employed even in a case where drone 30' is capable of moving on land.

### [Operation Example]

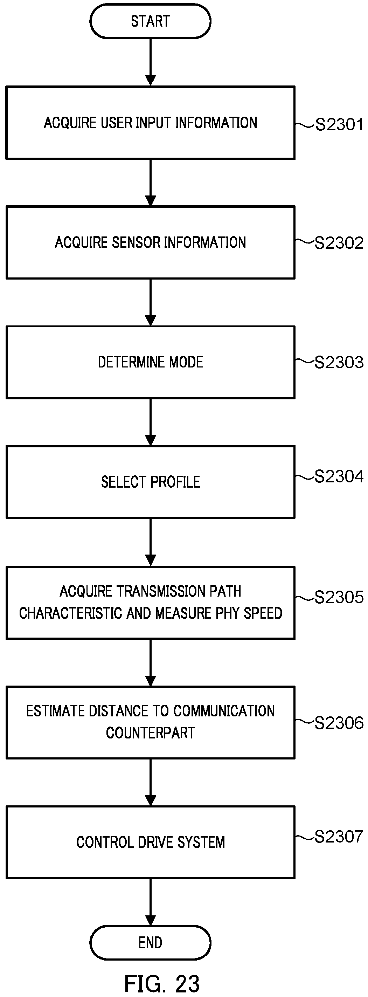

Next, an operation example of drone 30' will be described with reference to FIG 23. The processing illustrated in FIG. 23 may be started, for example, when drone 30' is activated.

In step S2301, user input information acquirer 311 acquires user input information from the user.

In step S2302, sensor information acquirer 312 acquires sensor information from sensor 207.

In step S2303, mode determiner 313 determines the mode related to the operating environment of drone 30'. Mode determiner 313 determines the mode as described with reference to FIGS. 20 to 22, for example.

In step S2304, profile selector 314 selects, from a plurality of profiles, the profile corresponding to the mode determined in step S2303. For example, in a case where the determined mode is the midair mode, a profile as illustrated in (A) in FIG. 19 is selected; in a case where the determined mode is the fresh water mode, a profile as illustrated in (B) in FIG. 19 is selected; and in a case where the determined mode is the sea water mode, a profile as illustrated in (C) in FIG. 19 is selected.

In step S2305, measurer 306 acquires a transmission path characteristic between drone 30' and the communication counterpart, and measures the PHY speed. The acquisition of the transmission path characteristic and the measurement of the PHY speed may be performed as described in Embodiment 1. For example, in a case where the AGC value is equal to or less than a threshold, control for decreasing the transmission power may be performed.

In step S2306, distance estimator 307 estimates the distance between drone 30' and the communication counterpart by using the profile selected in step S2304.

In step S2307, movement controller 310 controls drive system 204 (for example, a propeller or the like in the case of the midair mode; and a screw or the like in the case of the fresh water mode or the sea water mode) corresponding to the mode determined in step S2303, and causes drone 30' to move or stop. Note that, the descriptions in Embodiment 1 may be applied to the conditions for drone 30' to move, the determination of the direction in which drone 30' moves, the conditions for drone 30' to stop, and the like.

Note that, in a case where a movement of a predetermined value or more is performed (for example, in a case where a movement of a predetermined value or more is performed based on position information utilizing GPS, in a case where the rotational speed of a motor exceeds a threshold, or the like) after step S2307, the processing illustrated in FIG. 23 may be performed again from step S2301 or S2302.

Further, each time sensor information on a value to which the immediately preceding value has changed by a threshold or more is acquired, the processing illustrated in FIG. 23 may be performed again from step S2301, S2302 or S2303.

Although an example in which the mode determination is performed based on the user input information and/or the sensor information has been described above, the mode determination may alternatively or additionally be performed based on other information. For example, the mode determination may be performed based on the user input information, the sensor information and/or communication information from an external apparatus.

Although an example in which the mode is determined by the processing illustrated in FIG. 23 being performed when drone 30' is activated has been described above, the mode determination may not be performed when drone 30' is activated. For example, when drone 30' is activated, the immediately preceding mode in which drone 30' has been last turned off may be utilized.

Although an example in which the user input information is information on the mode itself has been described above, the user input information may be information on conditions for determining the mode.

The functional processor of CPU 201 described in the present embodiment may be integrated with another functional processor or may be divided into two or more sub-functional processors as appropriate. Further, the order of steps illustrated in flowcharts or the like described in the present embodiment is not limited to the illustrated order.

### <Effects in Embodiment 2>

Drone 30' capable of moving through a plurality of media such as air, sea water, and fresh water performs wireless communication with an external apparatus such as base unit 10, aircraft parking apron 20 and other drone 30' via short-distance antenna 210 and short-distance wireless communication circuitry 211. CPU 201 (measurer 306) of drone 30' acquires a transmission path characteristic (the SN ratio, the tone map, the PHY speed, or the like) between drone 30' and the external apparatus by transmitting and receiving signals to and from the external apparatus. CPU 201 (profile selector 314) of drone 30' selects, from a plurality of profiles corresponding to the plurality of different media, respectively, the profile corresponding to a medium in which drone 30' is present. CPU 201 (distance estimator 307) of drone 30' estimates the distance between drone 30' and the external apparatus based on the acquired transmission path characteristic and the selected profile. Thus, since the distance between drone 30' and the external apparatus is estimated based on the profile corresponding to the medium in which drone 30' is present, drone 30' can suppress an error in distance estimation in any medium when moving through a plurality of different media.

### <Summary of Embodiment 2>

A moving body according to an exemplary embodiment of the present disclosure is a moving body capable of moving through a plurality of different media, and includes: a communicator that performs wireless communication with an external apparatus; an index acquirer that acquires a transmission path characteristic between the moving body and the external apparatus; a selector that selects a profile corresponding to, among the plurality of different media, a medium in which the moving body is present; and a distance estimator that estimates a distance between the moving body and the external apparatus based on the transmission path characteristic and the profile.

The present moving body further includes: a sensor that measures a surrounding environment state of the moving body; and a determiner that determines, based on the surrounding environment state, the medium in which the moving body is present.

In the present moving body, the plurality of different media includes air, sea water, and fresh water.

In the present moving body, the sensor includes a salinity sensor, and in a case where a salinity measured by the salinity sensor is equal to or greater than a threshold, the determiner determines that the medium in which the moving body is present is the sea water.

In the present moving body, the profile associates the transmission path characteristic with a distance between communication apparatuses in the medium in which the moving body is present.

A distance estimation method according to an exemplary embodiment of the present disclosure includes: acquiring, by a moving body capable of moving through a plurality of different media and performing wireless communication with an external apparatus, a transmission path characteristic between the moving body and the external apparatus; selecting, by the moving body, a profile corresponding to, among the plurality of different media, a medium in which the moving body is present; and estimating, by the moving body, a distance between the moving body and the external apparatus based on the transmission path characteristic and the profile.

In the embodiments described above, the notation "... processor", "... -er", "... -or" or "... -ar" used for each component may be replaced with another notation such as "... circuitry", "... assembly", "... device", "... unit" or "... module".

Although the embodiments have been described above with reference to the accompanying drawings, the present disclosure is not limited to such examples. It is obvious that a person skilled in the art can arrive at various variations and modifications within the scope described in the claims. It is understood that such variations and modifications also belong to the technical scope of the present disclosure. Further, components in the embodiments described above may be arbitrarily combined without departing from the spirit of the present disclosure.

The present disclosure can be realized by software, hardware, or software in cooperation with hardware. Each functional block used in the description of each embodiment described above can be partly or entirely realized by an LSI such as an integrated circuit, and each process described in the each embodiment may be controlled partly or entirely by the same LSI or a combination of LSIs. The LSI may be individually formed as chips, or one chip may be formed so as to include a part or all of the functional blocks. The LSI may include a data input and output coupled thereto. The LSI here may be referred to as an IC, a system LSI, a super LSI, or an ultra LSI depending on a difference in the degree of integration.

However, the technique of implementing an integrated circuit is not limited to the LSI and may be realized by using a dedicated circuit, a general-purpose processor, or a special-purpose processor. In addition, a FPGA (Field Programmable Gate Array) that can be programmed after the manufacture of the LSI or a reconfigurable processor in which the connections and the settings of circuit cells disposed inside the LSI can be reconfigured may be used. The present disclosure can be realized as digital processing or analogue processing.

If future integrated circuit technology replaces LSIs as a result of the advancement of semiconductor technology or other derivative technology, the functional blocks could be integrated using the future integrated circuit technology. Biotechnology can also be applied.

The present disclosure can be realized by any kind of apparatus, device or system having a function of communication, which is referred to as a communication apparatus. The communication apparatus may comprise a transceiver and processing/control circuitry. The transceiver may comprise and/or function as a receiver and a transmitter. The transceiver, as the transmitter and receiver, may include an RF (radio frequency) module including amplifiers, RF modulators/demodulators and the like, and one or more antennas. Some non-limiting examples of such a communication apparatus include a phone (e.g., cellular (cell) phone, smart phone), a tablet, a personal computer (PC) (e.g., laptop, desktop, netbook), a camera (e.g., digital still/video camera), a digital player (digital audio/video player), a wearable device (e.g., wearable camera, smart watch, tracking device), a game console, a digital book reader, a telehealth/telemedicine (remote health and medicine) device, and a vehicle providing communication functionality (e.g., automotive, airplane, ship), and various combinations thereof.

The communication apparatus is not limited to be portable or movable, and may also include any kind of apparatus, device or system being non-portable or stationary, such as a smart home device (e.g., an appliance, lighting, smart meter, control panel), a vending machine, and any other "things" in a network of an "Internet of Things (IoT)".

The communication may include exchanging data through, for example, a cellular system, a wireless LAN system, a satellite system, etc., and various combinations thereof.

The communication apparatus may comprise a device such as a controller or a sensor which is coupled to a communication device performing a function of communication described in the present disclosure. For example, the communication apparatus may comprise a controller or a sensor that generates control signals or data signals which are used by a communication device performing a communication function of the communication apparatus.

The communication apparatus also may include an infrastructure facility, such as a base station, an access point, and any other apparatus, device or system that communicates with or controls apparatuses such as those in the above non-limiting examples.

The disclosure of Japanese Patent Application No. 2022-099655, filed on June 21, 2022, including the specification, drawings and abstract, is incorporated herein by reference in its entirety.

### Industrial Applicability

The present disclosure is useful for distance estimation techniques.

### Reference Signs List

1 Wireless power/data transmission system
10 Power source/communication equipment
20 Aircraft parking apron
30 Underwater drone
201 CPU
202 Memory
203 UI
204 Drive system
205 GPS receiver
206 Camera
207 Sensor
208 Long-distance antenna
209 Long-distance wireless communication circuitry
210 Short-distance antenna
211 Short-distance wireless communication circuitry
212 Power reception antenna
213 Power reception circuitry
214 Battery
215 Power source circuitry
301 Packet analyzer
302 Authentication processor
303 Link cost calculator
304 Topology manager
305 Packet generator
306 Measurer
307 Distance estimator
308 Transmission power determiner
309 AGC value confirmer
310 Movement controller
311 User input information acquirer
312 Sensor information acquirer
313 Mode determiner
314 Profile selector

## Claims

1. A moving body capable of moving through a plurality of different media, the moving body comprising:
a communicator that performs wireless communication with an external apparatus;
an index acquirer that acquires a transmission path characteristic between the moving body and the external apparatus;
a selector that selects a profile corresponding to, among the plurality of different media, a medium in which the moving body is present; and
a distance estimator that estimates a distance between the moving body and the external apparatus based on the transmission path characteristic and the profile.

2. The moving body according to claim 1, further comprising:
a sensor that measures a surrounding environment state of the moving body; and
a determiner that determines, based on the surrounding environment state, the medium in which the moving body is present.

3. The moving body according to claim 2, wherein
the plurality of different media includes air, sea water, and fresh water.

4. The moving body according to claim 3, wherein
the sensor includes a salinity sensor, and
in a case where a salinity measured by the salinity sensor is equal to or greater than a threshold, the determiner determines that the medium in which the moving body is present is the sea water.

5. The moving body according to claim 1, wherein
the profile associates the transmission path characteristic with a distance between communication apparatuses in the medium in which the moving body is present.

6. A distance estimation method, comprising:
acquiring, by a moving body capable of moving through a plurality of different media and performing wireless communication with an external apparatus, a transmission path characteristic between the moving body and the external apparatus;
selecting, by the moving body, a profile corresponding to, among the plurality of different media, a medium in which the moving body is present; and
estimating, by the moving body, a distance between the moving body and the external apparatus based on the transmission path characteristic and the profile.
